# EUROPEAN PATENT APPLICATION

(11) **EP 4 793 963 A1**
(43) Date of publication of application: **19.08.2026**
(21) Application number: 24876923.4
(22) Date of filing: 01.08.2024
(51) Int. Cl.: G16H 30/20

(54) **INFORMATION PROCESSING DEVICE, ANALYSIS DEVICE, INFORMATION PROCESSING METHOD, AND INFORMATION PROCESSING PROGRAM**

(30) Priority: 13.10.2023 JP 2023177846
(71) Applicant: ARKRAY, Inc., Kyoto-shi, Kyoto 601-8045 (JP)
(72) Inventor: ISHIBE, Daisuke, Kyoto-shi, Kyoto 602-0008 (JP)
(74) Representative: MFG Patentanwälte Meyer-Wildhagen Meggle-Freund Gerhard PartG mbB
(86) International application number: PCT/JP2024/027596
(87) International publication number: WO 2025/079322

(57) **Abstract**

An information processing device includes an acquisition unit, an imparting unit, a classification unit, an association unit, a reception unit, a grouping unit and a display control unit. The acquisition unit acquires material component images in which material components contained in a specimen are captured. The imparting unit imparts identification information for identifying the material component images to the material component images. The classification unit classifies the material component images based on types of the material components captured in the material component images. The association unit associates classifications of the material components with the material component images to which the identification information has been imparted. The reception unit receives, from a user, a designation of a classification among the classifications associated with the material component images. The grouping unit divides out the material component images associated with the designated classification into groups one by one in order. The display control unit performs control to display at a display unit, for each of the divided groups, a material component image associated with the designated classification.

## Description

### TECHNICAL FIELD

The present disclosure relates to an information processing device, an analysis device, an information processing method, and an information processing program.

### BACKGROUND ART

A urine analysis device that analyzes material components in urine is recited in, for example, Japanese Patent Application Laid-Open (JP-A) No. 2018-100976. This urine analysis device is equipped with an inspection device, which is a sedimentation apparatus that classifies and measures material components contained in a urine specimen; an imaging device that extracts cell images from photographic images capturing the urine specimen, automatically classifies types of material components contained in the cell images by image processing of the cell images, and sends the cell images and classification results to a management device; and the management device, which displays measurement results from the inspection device and imaging results from the imaging device at a display unit. The display unit displays cell images classified by the material components and displays a number of cell images for each material component.

### SUMMARY OF INVENTION

### TECHNICAL PROBLEM

The urine analysis device recited in JP-A No. 2018-100976 classifies material component images by automatic analysis and a user (for example, a medical technologist or the like) may examine the classified material component images. In this situation, the user may be examining large quantities of images in which, principally, similar shapes of bacteria, crystals, white blood cells, red blood cells, fungi and the like are captured. Examination of images one by one by a user is not efficient. Therefore, methods for enabling efficient examination are desired.

The present disclosure is made in consideration of the matter described above. An object of the present disclosure is to provide an information processing device, an analysis device, an information processing method, and an information processing program with which a user may examine classification results of material component images efficiently.

### SOLUTION TO PROBLEM

In order to achieve the object described above, an information processing device according to an aspect of the present disclosure includes: an acquisition unit that acquires material component images in which material components contained in a specimen are captured; a classification unit that classifies the material component images based on types of the material components captured in the material component images; a reception unit that receives a designation of a classification from a user; a grouping unit that divides out the material component images that are classified with the classification designated at the reception unit, into groups one by one, in order, the grouping unit: sequentially reading the material component images classified with the designated classification, memorizing a material component image that is read first as a representative image of a first group of the classification, comparing a material component image that is read next with the representative image of the first group, in a case in which this material component image and the representative image attain a predetermined similarity, memorizing this material component image in the first group, in a case in which this material component image and the representative image are not similar, memorizing this material component image as a representative image of a new group that is separate from the first group, comparing a material component image that is sequentially read third or subsequently with the memorized representative image of each group, in a case in which this material component image is similar to any of the representative images, grouping this material component image with a group whose representative image is similar, and in a case in which this material component image is not similar to any of the representative images, memorizing this material component image as a representative image of a new group; and a display control unit that performs control to display, at a display unit the representative image of the material component images in each group divided by the grouping unit.

Further, in order to achieve the object described above, an analysis device according to an aspect of the present disclosure includes: a flow cell through which a specimen containing material components flows; an imaging unit that images the specimen flowing through the flow cell; the information processing device, which is connected to the imaging unit; and a display unit connected to the information processing device.

Further, in order to achieve the object described above, an information processing method according to an aspect of the present disclosure includes a computer executing processing including: acquiring material component images in which material components contained in a specimen are captured; classifying the material component images based on types of the material components captured in the material component images; receiving a designation of a classification from a user; dividing out the material component images that are classified with the designated classification into groups one by one, in order, the dividing out including: sequentially reading the material component images classified with the designated classification, memorizing a material component image that is read first as a representative image of a first group of the classification, comparing a material component image that is read next with the representative image of the first group, in a case in which this material component image and the representative image attain a predetermined similarity, memorizing this material component image in the first group, in a case in which this material component image and the representative image are not similar, memorizing this material component image as a representative image of a new group that is separate from the first group, comparing a material component image that is sequentially read third or subsequently with the memorized representative image of each group, in a case in which this material component image is similar to any of the representative images, grouping this material component image with a group whose representative image is similar, and in a case in which this material component image is not similar to any of the representative images, memorizing this material component image as a representative image of a new group; and performing control to display at a display unit, the representative image of the material component images in each divided group.

Further yet, in order to achieve the object described above, an information processing program according to an aspect of the present disclosure causes a computer to execute processing including: acquiring material component images in which material components contained in a specimen are captured; classifying the material component images based on types of the material components captured in the material component images; receiving a designation of a classification from a user; dividing out the material component images that are classified with the designated classification into groups one by one, in order, the dividing out including: sequentially reading the material component images classified with the designated classification, memorizing a material component image that is read first as a representative image of a first group of the classification, comparing a material component image that is read next with the representative image of the first group, in a case in which this material component image and the representative image attain a predetermined similarity, memorizing this material component image in the first group, in a case in which this material component image and the representative image are not similar, memorizing this material component image as a representative image of a new group that is separate from the first group, comparing a material component image that is sequentially read third or subsequently with the memorized representative image of each group, in a case in which this material component image is similar to any of the representative images, grouping this material component image with a group whose representative image is similar, and in a case in which this material component image is not similar to any of the representative images, memorizing this material component image as a representative image of a new group; and performing control to display at a display unit, the representative image of the material component images in each divided group.

### ADVANTAGEOUS EFFECTS OF INVENTION

As described above, according to the present disclosure, an effect is provided in that a user may examine classification results of material component images efficiently.

### BRIEF DESCRIPTION OF DRAWINGS

Fig. 1 is a perspective view showing some of the structures of a urine material component analysis device according to a first exemplary embodiment.
Fig. 2 is a side view showing an example of the structures of the urine material component analysis device according to the first exemplary embodiment.
Fig. 3 is a block diagram showing an example of electronic structures of an information processing system according to the first exemplary embodiment.
Fig. 4 is a block diagram showing an example of functional structures of an information processing device according to the first exemplary embodiment.
Fig. 5 is views supporting a description of grouping processing according to the exemplary embodiment.
Fig. 6 is a flowchart showing an example of a flow of measurement processing that is executed by the information processing device when a specimen measurement instruction is received from a user.
Fig. 7 is a view showing an example of a material component image according to the exemplary embodiment.
Fig. 8 is a view showing an example of an inspection results screen displaying urine material component concentrations and urine qualitative inspection results.
Fig. 9 is a flowchart showing an example of a flow of grouping processing of material component images relating to step S90 in Fig. 6.
Fig. 10 is a view showing an example of a material component image display screen according to the exemplary embodiment.
Fig. 11 is a flowchart, continuing from Fig. 9, showing the example of the flow of grouping processing of material component images relating to step S90 in Fig. 6.
Fig. 12 is a view showing an example of the material component image display screen according to the exemplary embodiment in a state in which representative images of groups and information based on image counts of the groups are displayed.
Fig. 13 is a flowchart, continuing from Fig. 11, showing the example of the flow of grouping processing of material component images relating to step S90 in Fig. 6.
Fig. 14 is a view showing an example of the material component image display screen according to the exemplary embodiment in a state in which material component images in a group are displayed.
Fig. 15 is a block diagram showing an example of electronic structures of an information processing system according to a second exemplary embodiment.

### DETAILED DESCRIPTION

Below, examples of modes embodying the technology of the present disclosure are described in detail with reference to the attached drawings. Structural elements and processes that are responsible for the same operations and functions may be assigned the same reference symbols in all the drawings and duplicative descriptions thereof may be omitted as appropriate. The drawings are schematically illustrated only to an extent required to enable proper understanding of the present disclosure. Therefore, the technology of the present disclosure is not limited only to the illustrated examples. In the present exemplary embodiments, descriptions of structures. peripheral structures and the like that are not directly related to the present disclosure may be omitted.

### = First Exemplary Embodiment =

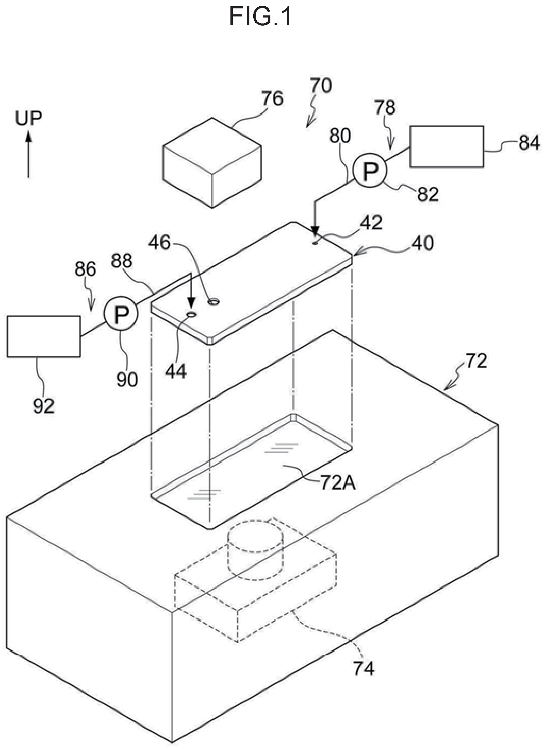

Fig. 1 is a perspective view showing some of the structures of a urine material component analysis device 70 according to a first exemplary embodiment. The urine material component analysis device 70 is an example of an analysis device.

As shown in Fig. 1, the urine material component analysis device 70 according to the present exemplary embodiment includes a flow cell 40, a casing 72, a camera 74 and a light source 76. The arrow UP that is shown in Fig. 1 indicates an upper side in a vertical direction of the urine material component analysis device 70.

The flow cell 40 according to the present exemplary embodiment is employed in, for example, a urine material components inspection (a urine sedimentation inspection) in which a urine specimen, which is an example of a specimen, is fed in together with a sheath fluid, material components in the urine specimen are captured by the camera 74, and various analyses are performed from shapes and the like of material components in captured images. The camera 74 is an example of an imaging unit. Plural types of material components are contained in the urine specimen. Types of material components may include, for example, red blood cells, white blood cells, epithelial cells, casts, bacteria and the like. In the present exemplary embodiment, a situation is described in which a urine specimen is employed as an example of a specimen and a urine material component inspection is performed to measure number concentrations of material components that are measurement objects in the urine, the respective measurement objects in the urine specimen being red blood cells, white blood cells, non-squamous epithelial cells, squamous epithelial cells, bacteria, crystals, yeasts, hyaline casts, other casts (also referred to as abnormal casts), mucus, sperm and white blood cell clumps. However, the present exemplary embodiment may also be employed for material component inspections of any fluid from the body, such as blood, cells, body fluids and the like.

The urine material component analysis device 70 is equipped with the casing 72 in which the flow cell 40 is disposed. A recess portion 72A into which the flow cell 40 is inserted is formed in the casing 72. A region of the casing 72 including the recess portion 72A is formed of a transparent member (of, for example, glass or the like). The camera 74 is provided inside the casing 72 at a position opposing the flow cell 40. The light source 76 is provided at the upper side of the casing 72, at a position opposing the camera 74 with the flow cell 40 therebetween. The camera 74 is disposed at a position that enables imaging of a specimen fluid flowing through the flow cell 40.

The urine material component analysis device 70 is equipped with a first supply apparatus 78 that supplies the specimen fluid to a specimen inlet port 42 of a specimen channel (not shown in the drawings) in the flow cell 40. The first supply apparatus 78 includes a supply channel 80, one end portion of which is connected to the specimen inlet port 42, and a pump 82 provided partway along the supply channel 80. A specimen reservoir portion (also referred to as a Spitz tube) 84 in which the specimen fluid is stored is disposed at another end portion of the supply channel 80. A barcode label is adhered to a side face of the Spitz tube 84. The barcode label shows a barcode representing a specimen ID for uniquely identifying the specimen in the Spitz tube 84.

The urine material component analysis device 70 is equipped with a second supply apparatus 86 that supplies the sheath fluid to a sheath inlet port 44 of a sheath channel (not shown in the drawings) in the flow cell 40. The second supply apparatus 86 includes a supply channel 88, one end portion of which is connected to the sheath inlet port 44, a pump 90 provided partway along the supply channel 88, and a tank 92 that is connected to another end portion of the supply channel 88 and stores the sheath fluid.

A discharge port 46 is provided at the flow cell 40, between the specimen inlet port 42 and the sheath inlet port 44. One end portion of a discharge tube (not shown in the drawings) is connected to the discharge port 46. Another end portion of this discharge tube is connected to a waste tank (not shown in the drawings). The flow cell 40 is equipped with a confluence unit (not shown in the drawings) in which the flows of the specimen fluid introduced through the specimen inlet port 42 and the sheath fluid introduced through the sheath inlet port 44 are joined. The joined fluids flow in a flow channel and material components in the specimen fluid are captured by the camera 74.

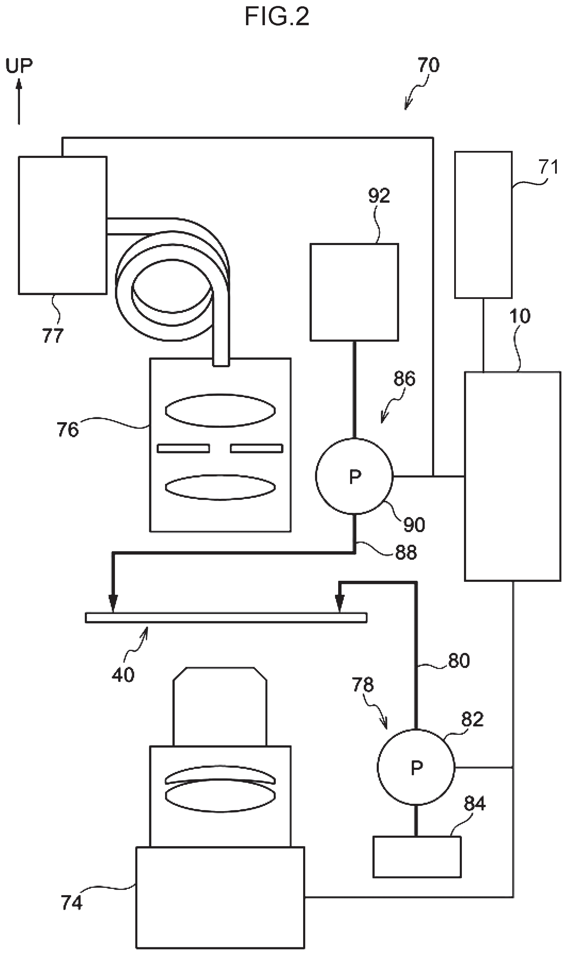

Fig. 2 is a side view showing an example of the structures of the urine material component analysis device 70 according to the first exemplary embodiment.

As shown in Fig. 2, the urine material component analysis device 70 according to the present exemplary embodiment is equipped with an information processing device 10. Similarly to Fig. 1, the arrow UP shown in Fig. 2 indicates the upper side in the vertical direction of the urine material component analysis device 70.

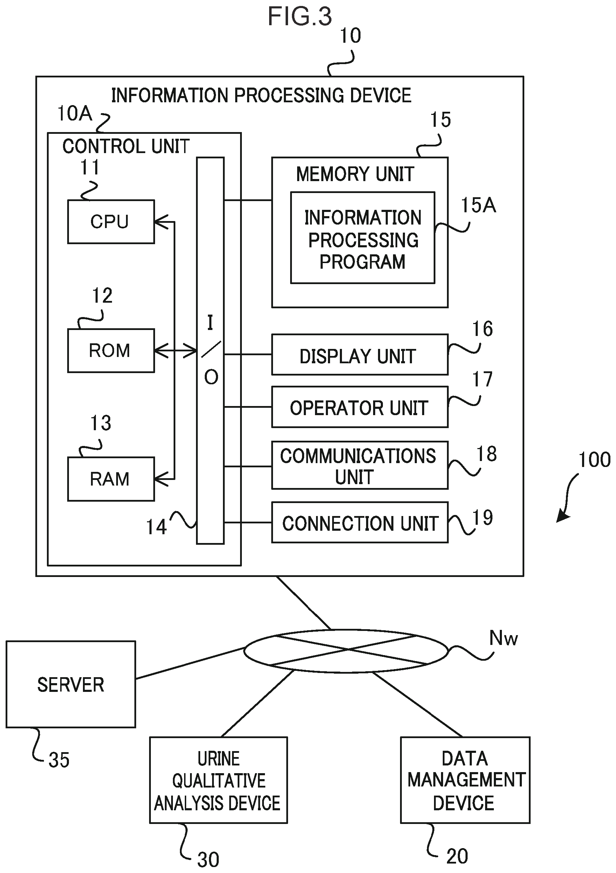

The information processing device 10 has functions of a control unit that controls the respective operations of the camera 74, a light source operation unit 77 that is electronically connected to the light source 76, the pump 82, and the pump 90. The information processing device 10 supplies pulse signals to the light source operation unit 77, causing the light source 76 to emit light at predetermined intervals. The information processing device 10 activates the pump 82 and controls flow amounts of the specimen fluid. The information processing device 10 also activates the pump 90 and controls flow amounts of the sheath fluid. Although not shown in the drawings, plural cameras 74 and optical systems that guide light to each of the cameras 74 may be provided. The optical systems are adjusted such that the focusing points of the cameras 74 are at different positions (depths) in the flow cell 40. That is, plural images that are focused on different depth positions at the same position in the horizontal plane may be captured simultaneously by the plural cameras 74. The captured images that are simultaneously captured are associated and memorized in a memory unit 15 shown in Fig. 3, which is described below. The meaning of the term "depth direction" as used herein is intended to include a direction orthogonal to the flow direction of the specimen fluid, which in Fig. 2 is the vertical direction. The focusing points differ in distance from a wall face at the side of the flow cell 40 that is closer to the cameras 74. The information processing device 10 is also connected to a barcode reader 71. The barcode reader 71 reads the specimen ID of the specimen from the barcode label adhered to the Spitz tube 84.

Fig. 3 is a block diagram showing an example of electronic structures of an information processing system 100 according to the first exemplary embodiment.

As shown in Fig. 3, the information processing system 100 according to the present exemplary embodiment includes the information processing device 10, a data management device 20, a urine qualitative analysis device 30 and a server 35. The information processing device 10 is connected to each of the data management device 20 and the server 35 via a network circuit Nw. The urine qualitative analysis device 30 is connected to the server 35 via the network circuit Nw. The urine qualitative analysis device 30 is linked with, for example, the urine material component analysis device 70.

The information processing device 10 includes a central processing unit (CPU) 11, read-only memory (ROM) 12, random access memory (RAM) 13, an input/output interface (I/O) 14, the memory unit 15, a display unit 16, an operator unit 17, a communications unit 18 and a connection unit 19. The CPU 11 may be a processor such as, for example, a graphics processing unit (GPU) or the like.

A general-purpose computer apparatus such as, for example, a personal computer (PC) or the like may be employed for the information processing device 10 according to the present exemplary embodiment. Alternatively, a portable computer apparatus such as a smartphone, a tablet terminal or the like may be employed for the information processing device 10. The information processing device 10 may be divided into plural units. For example, the information processing device 10 may be structured with a unit that controls a measurement system, which is the camera 74, the light source 76, the pump 82, the pump 90 and the like, and a unit that performs processing and analysis of images captured by the camera 74. The information processing device 10 may be externally connected to the urine material component analysis device 70.

A control unit 10A is constituted by the CPU 11, ROM 12, RAM 13 and input/output interface 14. The control unit 10A functions to control the measurement system that is, for example, the camera 74, light source 76, pump 82, pump 90 and the like, and functions to perform the processing and analysis of images captured by the camera 74. The respective parts of the CPU 11, ROM 12, RAM 13 and input/output interface 14 are connected to one another via a bus.

Functional units, including the memory unit 15, display unit 16, operator unit 17, communications unit 18 and connection unit 19, are connected to the input/output interface 14. These functional units are capable of reciprocal communications with the CPU 11 via the input/output interface 14.

The control unit 10A may be configured as a sub control unit that controls operations of parts of the information processing device 10, and may be configured as a portion of a main control unit that controls operations of the whole of the information processing device 10. An integrated circuit (IC) based on large-scale integration (LSI) or the like, or an IC chip set, may be employed for some or all of the blocks of the control unit 10A. Separate circuits may be employed for the above-mentioned blocks, and an integrated circuit may be employed for some or all of the blocks. The blocks may be provided integrally with one another, and some of the blocks may be provided separately. Portions of the respective blocks may be provided separately. Integration of the control unit 10A is not limited to LSI; a dedicated circuit or a general-purpose processor may be employed.

For example, a hard disk drive (HDD), solid state drive (SSD), flash memory or the like may be employed as the memory unit 15. An information processing program 15A for carrying out image classification processing according to the present exemplary embodiment is memorized at the memory unit 15. The information processing program 15A may be memorized at the ROM 12. External memory may be added to the memory unit 15 and may be subsequently expanded.

The information processing program 15A may, for example, be installed at the information processing device 10 in advance. The information processing program 15A may be memorized at a non-volatile, non-transitory memory medium, or may be distributed via the network circuit Nw and installed and upgraded at the information processing device 10 as appropriate. The non-volatile, non-transitory memory medium can be expected to be, for example, a compact disc read-only memory (CD-ROM), a magneto-optic disc, an HDD, a digital versatile disc read-only memory (DVD-ROM), a flash memory, a memory card or the like.

For example, a liquid crystal display (LCD), organic electroluminescent (EL) display or the like is employed for the display unit 16. The display unit 16 may integrally include a touch panel. A device for operational entries such as, for example, a keyboard and mouse or the like is provided at the operator unit 17. The display unit 16 and the operator unit 17 receive various instructions from a user of the information processing device 10.
The display unit 16 displays results of processing executed in accordance with instructions received from the user, and various kinds of information such as reports on processing and the like.

The communications unit 18 is connected to the network circuit Nw, which is the Internet, a local area network (LAN), a wide area network (WAN) or the like. The communications unit 18 enables communications with the data management device 20 via the network circuit Nw.

The connection unit 19 is connected with, for example, the barcode reader 71, the camera 74, the light source 76, the pump 82, the pump 90 and so forth. The measurement system that is the barcode reader 71, camera 74, light source 76, pump 82, pump 90 and the like is controlled by the control unit 10A described above. The connection unit 19 functions as an input port that inputs images outputted by the camera 74.

The information processing device 10 measures number concentrations of material components in the specimen from results of classification of material component images. When a repeat measurement of number concentrations is required, the information processing device 10 controls the communications unit 18 and sends material component images to the data management device 20 via the network circuit Nw. The data management device 20 performs a reclassification of the material component images received from the information processing device 10 and sends results of the reclassification to the information processing device 10.

The urine qualitative analysis device 30 is linked with the urine material component analysis device 70 via a specimen transport channel. The urine qualitative analysis device 30 is equipment for carrying out a urine qualitative inspection of the specimen. The urine qualitative inspection referred to herein encompasses, for example, a test in which urine is applied to a test paper known as TES-TAPE that reacts with a measurement object component in the urine specimen and changes color, the change in color is measured, and whether or not the measurement object component is present in the urine specimen is determined, and a test that measures a concentration of a measurement object component in the urine specimen. The urine qualitative analysis device 30 is equipped with the barcode reader 71 (see Fig. 2) for reading the specimen ID of a measurement object specimen from the barcode label adhered to the side face of the Spitz tube 84. The urine qualitative analysis device 30 links (associates) the urine qualitative inspection results of the urine specimen inspected by the urine qualitative analysis device 30 with the specimen ID of the urine specimen, and sends the same to the server 35 via the network circuit Nw. The specimen ID and urine qualitative inspection results linked with the specimen ID that are sent are memorized at the server 35. When an error occurs during a measurement of a urine specimen, the urine qualitative analysis device 30 links error information for the urine specimen with the specimen ID of the urine specimen and sends the same to the server 35 via the network circuit Nw.

The server 35 memorizes urine qualitative inspection results and error information sent from the urine qualitative analysis device 30. The information processing device 10 may acquire urine qualitative inspection results and error information relating to the urine qualitative analysis device 30 from the server 35.

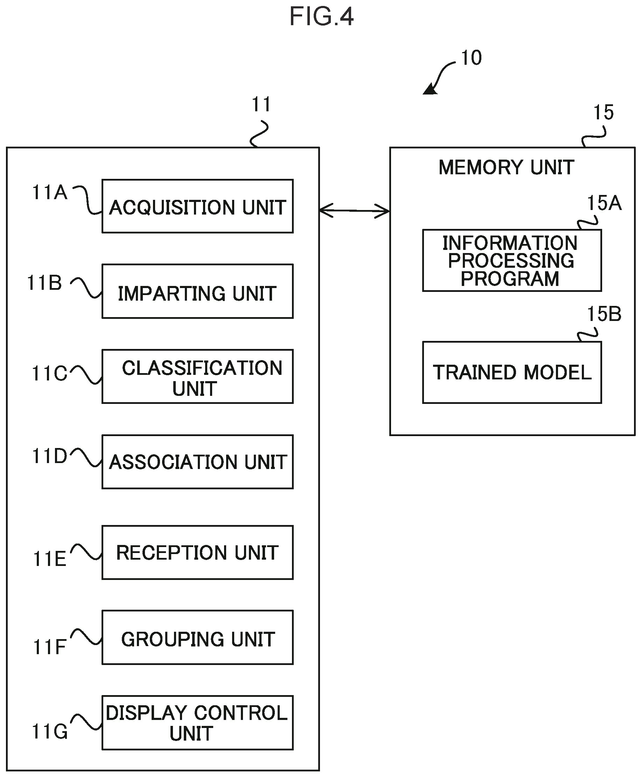

Now, functional structures of the information processing device 10 according to the first exemplary embodiment are described in specific terms with reference to Fig. 4.

The CPU 11 of the information processing device 10 according to the present exemplary embodiment writes the information processing program 15A memorized at the memory unit 15 to the RAM 13 and executes the information processing program 15A. As a result, the CPU 11 functions as the units shown in Fig. 4.

Fig. 4 is a block diagram showing an example of functional structures of the information processing device 10 according to the first exemplary embodiment.

As shown in Fig. 4, the CPU 11 according to the present exemplary embodiment functions as an acquisition unit 11A, an imparting unit 11B, a classification unit 11C, an association unit 11D, a reception unit 11E, a grouping unit 11F and a display control unit 11G.

A trained model 15B that is used for image classification processing is memorized at the memory unit 15. The trained model 15B is a model that is used for image classification processing by the classification unit 11C.

The acquisition unit 11A acquires material component images in which material components contained in the specimen are captured. More specifically, from plural images that are obtained by the camera 74 imaging the specimen flowing through the flow cell 40 (below referred to as "specimen images"), the acquisition unit 11A extracts plural types of material components contained in the specimen as the material component images, acquiring at least one extracted material component image. More specifically, the acquisition unit 11A uses various widely known technologies such as image processing, meaning binarization processing and edge detection, processes that employ machine learning, processes that employ pattern matching, and so forth to extract the material component images from the respective specimen images. One material component is contained in each of the respective material component images. That is, each material component image captures one material component. For example, 300 or 1000 of the specimen images are acquired from one specimen.

The imparting unit 11B imparts identification information for identifying the material component images to the material component images acquired by the acquisition unit 11A. The identification information is, for example, material component image IDs that are determined on the basis of the order in which the material component images are extracted from the specimen images.

The classification unit 11C classifies the material component images acquired by the acquisition unit 11A on the basis of types of the material components captured in the material component images. More specifically, the classification unit 11C classifies the material component images acquired by the acquisition unit 11A as detected components in each of predetermined classifications (for example, types of material component, sizes, shapes, the presence or absence of a nucleus, and so forth). Material component image groups classified in each of the predetermined classifications by the classification unit 11C are allotted to each specimen and temporarily stored in the memory unit 15. Methods that can be applied for classifying the material component images are various widely known technologies such as, for example, processes that employ machine learning, processes that employ pattern matching and the like. The material component image groups according to the present exemplary embodiment are classified by using, for example, the trained model 15B. The trained model 15B is a model that is generated by machine learning from training data obtained by associating material component images obtained previously with respective detected components of each of the predetermined classifications. That is, the training data is supervised data. The trained model 15B inputs material component images and outputs detected components in the respective predetermined classifications. A learning model that is used for carrying out the machine learning is, for example, a convolutional neural network (CNN) or the like. A method that is used for the machine learning is, for example, deep learning or the like. The material component image groups are referred to as material component images when individual material component images are being described.

Principal types of material components include, for example, red blood cells, white blood cells, non-squamous epithelial cells, squamous epithelial cells, bacteria, crystals, yeasts, hyaline casts, other casts, mucus, sperm, white blood cell clumps, and other material components (below referred to as unclassified) such as, for example, material components to which material bodies of different types are adhered. Red blood cells are denoted by "RBC", white blood cells are denoted by "WBC", non-squamous epithelial cells are denoted by "NSE", squamous epithelial cells are denoted by "SQEC", other casts are denoted by "NHC", and bacteria are denoted by "BACT". Furthermore, crystals are denoted by "CRYS", yeasts are denoted by "YST", hyaline casts are denoted by "HYAL", mucus is denoted by "MUCS", sperm is denoted by "SPRM", and white blood cell clumps are denoted by "WBCC". Material components other than red blood cells, white blood cells, non-squamous epithelial cells, squamous epithelial cells, bacteria, crystals, yeasts, hyaline casts, other casts, mucus, sperm and white blood cell clumps are denoted by "UNCL" (unclassified) or as other material components. That is, the detected components of each of the predetermined classifications detected by the classification unit 11C correspond to components that are defined as the respective material components and as unclassified.

When classifying the material component images, the classification unit 11C calculates compatibilities on the basis of the image classification method that is employed (for example, machine learning, pattern matching or the like). The classification unit 11C classifies a material component image in, for example, the classification with the highest compatibility. The meaning of the term "compatibility" as used herein is intended to include a classification precision of an image in classification results. For each of the predetermined classifications, an image is assigned a higher value when a proportion of matching with a correct image or pre-specified characteristic points is higher. When the correct image or characteristic points match completely, the compatibility is 100%. That is, a material component image with a relatively low compatibility would be regarded as having a high likelihood of not being assigned to a suitable classification. The compatibility may be represented as a relevance ratio.

The association unit 11D associates the classifications of the material components classified by the classification unit 11C with the material component images to which the material component image IDs are imparted by the imparting unit 11B. Thus, a material component image ID, a material component image and a material component classification are associated with one another. The association unit 11D also calculates number concentrations of the material components in the specimen on the basis of numbers (counts) of the material component images that are classified with each of the material component classifications.

The reception unit 11E receives, from a user, a designation of a classification among the classifications associated with the material component images by the association unit 11D.

The grouping unit 11F divides out the material component images associated with the classification designated by the reception unit 11E into groups one by one in order. This division into groups is referred to as grouping processing. The grouping unit 11F successively reads the material component images classified to the classification designated by the reception unit 11E. The grouping unit 11F memorizes the material component image that is read first in, for example, the memory unit 15 as a representative image of a first group of the classification. The grouping unit 11F compares the material component image that is read next with the representative image of the first group. If the two images attain a predetermined degree of similarity, the grouping unit 11F memorizes this material component image at, for example, the memory unit 15 as one of the first group. If the two images are not similar, the grouping unit 11F memorizes the material component image at, for example, the memory unit 15 as a representative image of a new group that is separate from the first group. For each material component image that is successively read third and subsequently, the grouping unit 11F compares the material component image with the memorized representative image of each group. If the material component image is similar to one of the representative images, the grouping unit 11F groups the material component image with a group. If the material component image is not similar to any of the representative images, the grouping unit 11F memorizes the material component image at, for example, the memory unit 15 as a representative image of a new group.

More specifically, a number of material component images associated with the designated classification may be represented by n, a processing count may be represented by N, a group type number of the groups associated with the material component image IDs associated with the designated classification may be represented by m, and a processing group count may be represented by M. The material component image IDs associated with the designated classification are arranged in sequence by a predetermined condition, and while the grouping unit 11F increments N from 1 to (n-1) in steps of 1 (increasing), the grouping unit 11F specifies the material component image corresponding with the material component image ID that is in each place (N+1) from the start of this sequence.

The grouping unit 11F increments M from 1 to m in steps of 1 and, in a sequence in which the material component image IDs associated with each M^{th} group are arranged by a predetermined condition, selects each material component image ID that is in first place.

When M is equal to m, for each of the material component images corresponding with the selected material component image IDs, the grouping unit 11F calculates a similarity between the respective material component image corresponding with the selected material component image ID and the specified material component image corresponding with the material component image ID that is in place (N+1). The similarity is calculated using, for example, widely known pattern (template) matching. The similarity may be calculated by comparing local characteristic quantities. It is desirable to normalize the two images being compared in advance such that the images may be matched even if even if there are brightness differences between the two images. A similarity according to the present exemplary embodiment is a numerical value representing a degree to which the two images are similar, with a higher numerical value indicating that the two images are more alike.

When the highest similarity among the calculated similarities is at least a threshold value, the grouping unit 11F divides out the specified material component image corresponding with the material component image ID that is in place (N+1) to the same group as the group to which the material component image presenting the highest similarity has been divided out. On the other hand, when the highest similarity among the calculated similarities is less than the threshold value, the grouping unit 11F divides out the specified material component image corresponding with the material component image ID that is in place (N+1) to an (m+1)^{th} group. The (m+1)^{th} group is a new group that is different from the m^{th} group.

For each of the groups divided by the grouping unit 11F, the display control unit 11G controls the display unit 16 to display the material component image corresponding with the material component image ID that is first in that group as a representative image.

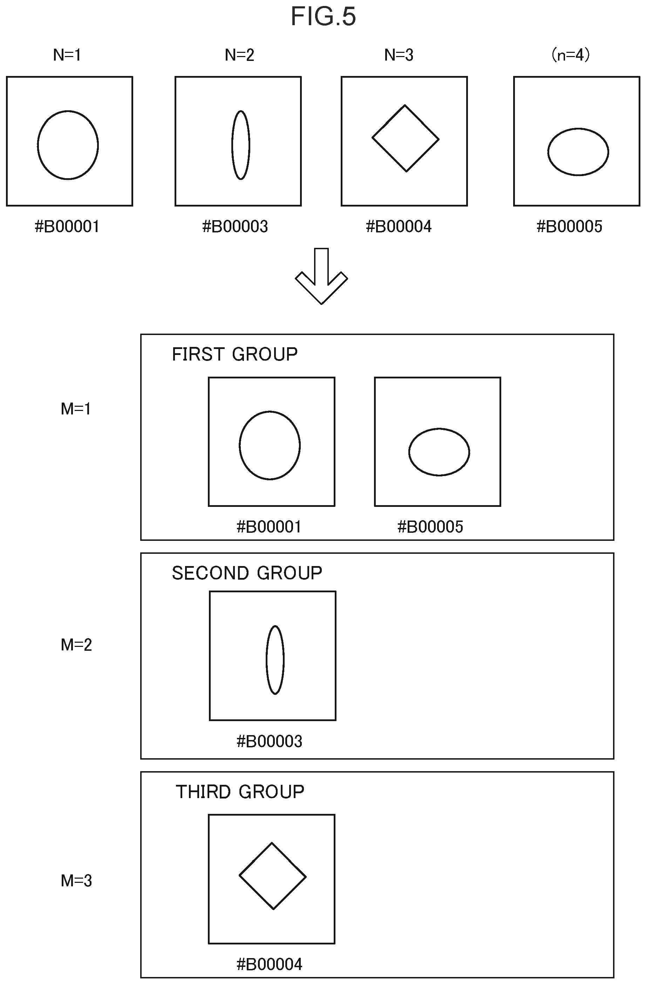

Fig. 5 is views supporting a description of the grouping processing according to the present exemplary embodiment. The example in Fig. 5 describes a situation in which the designated classification is red blood cells.

The material component images illustrated in Fig. 5 are, for example, images corresponding with the classification "red blood cells" and are assigned the material component image IDs "#B00001", "#B00003", "#B00004" and "#B00005". For simplicity of description, four of the material component images are illustrated but the description is similarly applicable to five or more material component images.

The predetermined condition when arranging the material component image IDs in sequence is, for example, arranging the material component image IDs in numerical order of the numerical part included in each material component image ID. The predetermined condition may be arranging the material component image IDs in reverse numerical order of the numerical parts, and may be arranging the material component image IDs randomly. Arrangement in alphabetical order is also applicable.

The grouping unit 11F counts the number n of the material component images. In the example in Fig. 5, n equals 4.

The grouping unit 11F divides out the material component image corresponding with the material component image ID that is first in the sequence of the material component image IDs arranged in numerical order to a first group. In the example in Fig. 5, the material component image corresponding with #B00001 is divided out to the first group.

The grouping unit 11F assigns the value 1 to the processing count N, and specifies the material component image corresponding with the material component image ID that is second (place N+1) from the start of the sequence of the material component image IDs arranged in numerical order. In the example in Fig. 5, the material component image corresponding with #B00003 is specified.

The grouping unit 11F counts the group type number m of groups associated with the material component image IDs that are associated with the designated classification. In the example in Fig. 5, because the first group exists at this time, m equals 1.

The grouping unit 11F assigns the value 1 to the processing group count M, and writes the material component image ID that is first in a sequence arranged in numerical order of the material component images IDs corresponding with the first (M^{th}) group to a comparison list (described below). In the example in Fig. 5, the grouping unit 11F selects #B00001 from the first group and writes #B00001 to the comparison list. The comparison list is stored at, for example, the memory unit 15.

Now M equals m equals 1. Accordingly, the grouping unit 11F calculates a similarity between the material component image corresponding with the material component image ID written to the comparison list and the specified material component image corresponding with the material component image ID that is in second place, and writes the calculated similarity to the comparison list. In the example in Fig. 5, a similarity between the material component image corresponding with #B00001 in the first group and the specified material component image corresponding with #B00003 is calculated, and this calculated similarity is written to the comparison list.

If the similarity written to the comparison list is at least the threshold value, the grouping unit 11F divides out the specified material component image corresponding with #B00003 to the first group. On the other hand, if the similarity written to the comparison list is less than the threshold value, the specified material component image corresponding with #B00003 is divided out to a second ((m+1)^{th}) group. In the example in Fig. 5, the similarity between the material component image corresponding with #B00001 in the first group and the specified material component image corresponding with #B00003 is less than the threshold value. Therefore, the specified material component image corresponding with #B00003 is divided out to the second group.

When the grouping unit 11F assigns the value N+1 to N (=1), N becomes 2 (less than n, which equals 4). Accordingly, the grouping unit 11F returns to processing that specifies the material component image corresponding with the material component image ID that is in place (N+1) from the start of the sequence of material component image IDs arranged in numerical order. In the example in Fig. 5, because (N+1) equals 3, the material component image corresponding with #B00004 is specified. The similarity that was written to the comparison list for the division to the second group is deleted.

Then, the grouping unit 11F counts the group type number m of groups associated with the material component image IDs that are associated with the designated classification. In the example in Fig. 5, because the first group and the second group exist at this time, m equals 2.

The grouping unit 11F assigns the value 1 to the processing group count M, and writes the material component image ID that is first in the sequence arranged in numerical order of the material component images IDs corresponding with the first (M^{th}) group to the comparison list. In the example in Fig. 5, the grouping unit 11F selects #B00001 from the first group and writes #B00001 to the comparison list. Here, because #B00001 is already written to the comparison list, the comparison list is overwritten.

Now M equals 1, less than m which is 2. Accordingly, the grouping unit 11F returns to the processing that assigns the value 2 (=M+1) to M, selects the material component image ID that is first in the sequence arranged in numerical order of the material component images IDs associated with the second (M^{th}) group, and writes this material component image ID to the comparison list. In the example in Fig. 5, the grouping unit 11F selects #B00003 from the second group and writes #B00003 to the comparison list.

When M equals m equals 2, for each material component image corresponding with a material component image ID written to the comparison list, the grouping unit 11F calculates a similarity between the respective material component image corresponding with the material component image ID written to the comparison list and the specified material component image corresponding with the material component image ID that is in third place, and writes the calculated similarity to the comparison list. In the example in Fig. 5, respective similarities are calculated between the specified material component image corresponding with #B00004 and each of the material component images corresponding with #B00001 from the first group and #B00003 from the second group, and the calculated similarities are written to the comparison list.

If the highest of the similarities written to the comparison list is at least the threshold value, the grouping unit 11F divides out the specified material component image corresponding with #B00004 to the same group as the group to which the material component image presenting the highest similarity has been divided out. On the other hand, if the highest of the similarities written to the comparison list is less than the threshold value, the specified material component image corresponding with #B00004 is divided out to a third ((m+1)^{th}) group. In the example in Fig. 5, the highest of the similarities written to the comparison list is less than the threshold value. Therefore, the specified material component image corresponding with #B00004 is divided out to the third group.

When the grouping unit 11F assigns the value N+1 to N (=2), N becomes 3 (less than n, which equals 4). Accordingly, the grouping unit 11F returns to processing that specifies the material component image corresponding with the material component image ID that is in place (N+1) from the start of the sequence of material component image IDs arranged in numerical order. In the example in Fig. 5, because (N+1) equals 4, the material component image corresponding with #B00005 is specified.

Then, the grouping unit 11F counts the group type number m of groups associated with the material component image IDs that are associated with the designated classification. In the example in Fig. 5, because the first group, the second group and the third group exist at this time , m equals 3.

The grouping unit 11F assigns the value 1 to the processing group count M, and writes the material component image ID that is first in the sequence arranged in numerical order of the material component images IDs corresponding with the first (M^{th}) group to the comparison list. In the example in Fig. 5, the grouping unit 11F selects #B00001 from the first group and writes #B00001 to the comparison list.

Because M equals 1, less than m which is 3, the grouping unit 11F returns to the processing that assigns the value 2 (=M+1) to M, selects the material component image ID that is first in the sequence arranged in numerical order of the material component images IDs associated with the second (M^{th}) group, and writes this material component image ID to the comparison list. In the example in Fig. 5, the grouping unit 11F selects #B00003 from the second group and writes #B00003 to the comparison list.

Because M equals 2, less than m which is 3, the grouping unit 11F returns to the processing that assigns the value 3 (=M+1) to M, selects the material component image ID that is first in the sequence arranged in numerical order of the material component images IDs associated with the third (M^{th}) group, and writes this material component image ID to the comparison list. In the example in Fig. 5, the grouping unit 11F selects #B00004 from the third group and writes #B00004 to the comparison list.

When M equals m equals 3, for each material component image corresponding with a material component image ID written to the comparison list, the grouping unit 11F calculates a similarity between the respective material component image corresponding with the material component image ID written in the comparison list and the specified material component image corresponding with the material component image ID that is in fourth place, and writes the calculated similarity to the comparison list. In the example in Fig. 5, respective similarities are calculated between the specified material component image corresponding with #B00005 and each of the material component images corresponding with #B00001 from the first group, #B00003 from the second group and #B00004 from the third group, and the calculated similarities are written to the comparison list.

If the highest of the similarities written to the comparison list is at least the threshold value, the grouping unit 11F divides out the specified material component image corresponding with #B00005 to the same group as the group to which the material component image presenting the highest similarity has been divided out. On the other hand, if the highest of the similarities written to the comparison list is less than the threshold value, the specified material component image corresponding with #B00005 is divided out to a fourth ((m+1)^{th}) group. In the example in Fig. 5, #B00001 presents the highest similarity of the similarities written to the comparison list, and this similarity is equal to or greater than the threshold value. Therefore, the specified material component image corresponding with #B00005 is divided out to the first group, the same group as #B00001.

When the grouping unit 11F assigns the value N+1 to N (=3), N becomes 4 (which equals n). Accordingly, the grouping unit 11F ends the grouping of the material component images associated with red blood cells.

### < Measurement processing by the information processing device 10 >

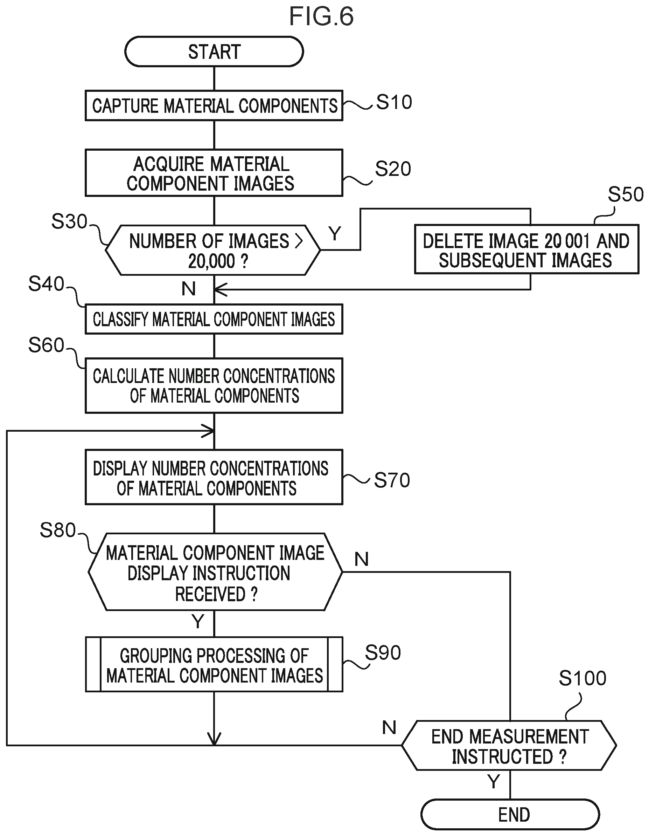

Now, operation of the information processing device 10 according to the present exemplary embodiment is described with reference to Fig. 6.

Fig. 6 is a flowchart showing an example of a flow of measurement processing that is executed by the information processing device 10 when a specimen measurement instruction is received from a user. The CPU 11 of the information processing device 10 reads the information processing program 15A memorized at the memory unit 15 and executes the measurement processing.

In step S10 of Fig. 6, the control unit 10A activates a conveyance unit (not shown in the drawings) and conveys the Spitz tube 84 containing a specimen, which has been placed at a predetermined position of the conveyance unit, to a specimen collection position. The barcode reader 71 (see Fig. 2) is mounted at the specimen collection position. The control unit 10A uses the barcode reader 71 to read the barcode label adhered to the side face of the Spitz tube 84. The barcode label shows a barcode representing, for example, a specimen ID for uniquely identifying the specimen. By reading the barcode label, the control unit 10A acquires the specimen ID of the specimen to be measured.

A distal end of the supply channel 80 (a distal end at the opposite side of the supply channel 80 from a side thereof at which a distal end is connected with the specimen inlet port 42) is disposed above an opening portion of the Spitz tube 84 that has been conveyed to the specimen collection position. The control unit 10A controls an actuator (not shown in the drawings) that moves the supply channel 80 in the vertical direction of the urine material component analysis device 70, to lower the distal end of the supply channel 80 through the opening portion into the Spitz tube 84. The control unit 10A lowers the distal end of the supply channel 80 to a position where the distal end of the supply channel 80 has reached the specimen, and then activates the pump 82. As a result, the specimen in the Spitz tube 84 is fed in to the flow cell 40 through the specimen inlet port 42 at a predetermined flow rate, and a regulated volume of the specimen flows into the flow cell 40.

Together with the activation of the pump 82, the control unit 10A activates the pump 90. As a result, the sheath fluid stored in the tank 92 is fed in to the flow cell 40 through the sheath inlet port 44 at a predetermined flow rate, and the sheath fluid joins together with the specimen in the flow cell 40.

The control unit 10A controls the camera 74 to capture images of the specimen in the flow cell 40 and memorizes the captured specimen images at, for example, the memory unit 15. A number of specimen images to be captured is not limited. For example, the control unit 10A captures images of the specimen in a number that is memorized in advance at the memory unit 15. A user may change the number of specimen images to be captured that is memorized at the memory unit 15, via the operator unit 17.

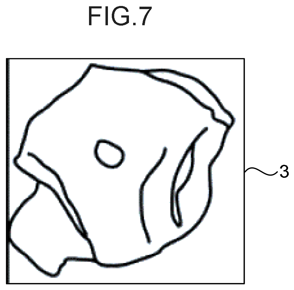

In step S20, the acquisition unit 11A acquires material component images. More specifically, various types of material components are contained in the captured specimen images. Accordingly, the acquisition unit 11A extracts images of the respective material components contained in the specimen images. That is, the acquisition unit 11A extracts a material component image 3, as illustrated by the example in Fig. 7, for each of the material components.

Fig. 7 depicts an example of the material component images 3 according to the present exemplary embodiment. Each material component image 3 is an image with a rectangular shape containing the whole of a material component. Accordingly, the size of each material component image 3 is altered in accordance with the size of the material component.

The imparting unit 11B imparts a material component image ID to each material component image 3 extracted from the specimen images. The material component image ID is an identifier for uniquely identifying each material component image 3, and can be used as, for example, a filename of the material component image 3. The imparting unit 11B generates a classification list that associates each material component image 3 with the specimen ID of the specimen from which the material component image 3 has been captured, and memorizes the classification list at, for example, the memory unit 15. Table 1 depicts an example of the classification list. Because the respective material component images 3 are images obtained from the same specimen, as illustrated in Table 1, the same specimen ID is associated with the respective material component image IDs.

**Table 1**

| Specimen ID | Material component image ID |
|---|---|
| #A0002 | #B00001 |
| #A0002 | |
| #A0002 | |
| : | : |
| #A0002 | #B17904 |
| #A0002 | #B17905 |

In step S30 of Fig. 6, the control unit 10A makes a determination as to whether the number of images is greater than 20,000. When the control unit 10A determines that the number of images is not more than 20,000 (the result of the determination is negative), the information processing device 10 proceeds to step S40, and when the control unit 10A determines that the number of images is more than 20,000 (the result of the determination is affirmative), the information processing device 10 proceeds to step S50.

In step S40, the classification unit 11C uses the trained model 15B memorized in advance at the memory unit 15 to classify the respective material component images 3 as the types of material component.

As mentioned above, the trained model 15B is an example of a classification model for the material component images 3 that has been generated by machine learning using training data in which the inputs are material component images 3, for which the types of material components the material component images 3 contain are already known, and the outputs are the types of material components contained in those material component images 3. A number of nodes in the output layer of the trained model 15B matches the number of types of material components that can be classified by the information processing device 10. The nodes in the output layer of the memory unit 15 are associated one-to-one with the respective types of material component.

When each material component image 3 is inputted to the trained model 15B, the trained model 15B outputs compatibilities from the respective nodes of the output layer. Each node of the output layer is associated with a type of material component. Therefore, the classification unit 11C classifies the type of material component contained in the material component image 3 inputted to the trained model 15B as the type of material component associated with the node of the output layer that outputs the highest compatibility. In this manner, the classification unit 11C sequentially inputs all of the material component images 3 extracted from the specimen images to the trained model 15B, and thus classifies the respective material component images 3 contained in the specimen images to the types of material component. That is, by classifying the material component images into material component images capturing measurement object material components and material component images capturing material components other than the measurement object material components (other material components), the classification unit 11C may identify material component images in which the measurement object material components are captured among the material component images.

The association unit 11D associates the material component image IDs in the classification list illustrated in Table 1 with the types of material components contained in the material component images 3, which are classified using the trained model 15B and represented by the material component image IDs. Table 2 shows an example of a classification list in which types of material components are associated. The values in the classification field of the classification list in Table 2 need not necessarily be the names of material components but may be symbols representing the material component names. The classification list associating types of material components with the respective material component image IDs is an example of classification results of material component images.

**Table 2**

| Specimen ID | Material component image ID | Classification |
|---|---|---|
| #A0002 | #B0001 | Red blood cells |
| #A0002 | #B0002 | White blood cells |
| #A0002 | #B0003 | Red blood cells |
| #A0002 | #B0004 | Red blood cells |
| #A0002 | #B0005 | Red blood cells |
| : | : | : |
| #A0002 | #B17904 | Squamous epithelial cells |
| #A0002 | #B17905 | Bacteria |

In step S50, the control unit 10A deletes material component images in places 20,001 and greater from the memory unit 15; then the CPU 11 proceeds to step S40.

In step S60, the association unit 11D refers to the classification list obtained by the processing of step S40, as illustrated in Table 2, and calculates number concentrations of the material components contained in the specimen on the basis of counts of the material component images 3 that are classified to each type of material component. The meaning of the term "number concentration" of a material component as used herein encompasses, for example, an index that represents a concentration of the material component contained in the specimen according to a count of the material component contained in a pre-specified unit volume, such as 1 µL.

More specifically, the association unit 11D uses concentration expressions memorized in advance at the memory unit 15 to calculate the respective number concentrations of the material components contained in the specimen. Table 3 shows examples of respective concentration expressions for the material components.

**Table 3**

| Classification | Image count | Concentration expression | Number concentration (counts/µL) |
|---|---|---|---|
| Red blood cells | X1 | y=a1*x+b1 | Y1 |
| White blood cells | X2 | y=a2*x+b2 | Y2 |
| Non-squamous epithelial cells | X3 | y=a3*x+b3 | Y3 |
| Squamous epithelial cells | X4 | y=a4*x+b4 | Y4 |
| Other casts | X5 | y=a5*x+b5 | Y5 |
| Bacteria | X6 | y=a6*x+b6 | Y6 |
| Crystals | X7 | y=a7*x+b7 | Y7 |
| Yeasts | X8 | y=a8*x+b8 | Y8 |
| Hyaline casts | X9 | y=a9*x+b9 | Y9 |
| Mucus | X10 | y=a10*x+b10 | Y10 |
| Sperm | X11 | y=a11*x+b11 | Y11 |
| White blood cell clumps | X12 | y=a12*x+b12 | Y12 |
| Other material components | X13 | y=a13*x+b13 | Y13 |

In the concentration expressions illustrated in Fig. 3, the operator "*" is an operator representing multiplication. The number concentration "y" of each material component is expressed by, for example, a first-order function that uses the number of the material component images 3 for that type of material component as an explanatory variable "x". The symbol "an" in each concentration expression (in which "n" is an integer) is a gradient determined for that type of material component, and the symbol "bn" is an intercept determined for that type of material component. The symbol "Xn" for each type n of the material components is the count of the material component images 3, and the symbol "Yn" is the number concentration of each type n of the material components. The concentration expression for each material component is a calculation expression that is prepared in advance by experimentation, computer simulation or the like to find a relationship between the number of the material component images 3 capturing the material component contained in a prescribed volume and the number concentration of the material component. The concentration expressions are memorized at the memory unit 15.

The concentration expressions shown in Table 3 are examples. Concentration expressions for the material components are not limited to first-order functions. Table 3 shows concentration expressions corresponding to 13 types of material components, but this number of classifications of material components by the information processing device 10 is an example.

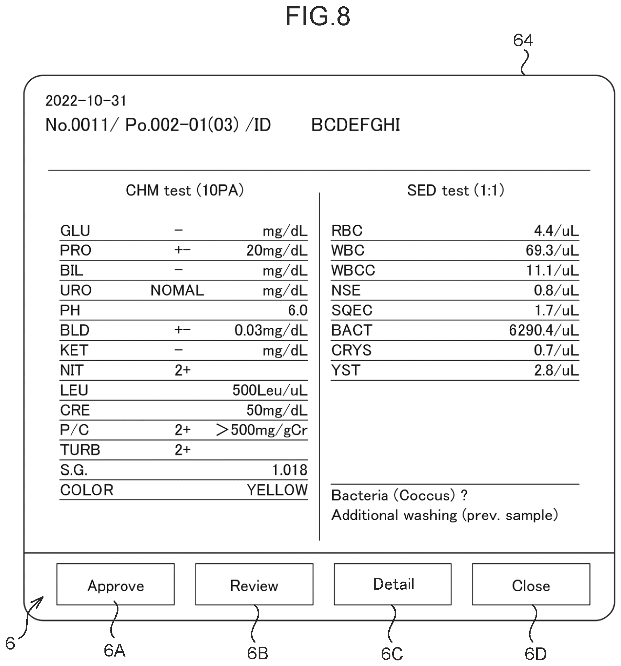

In step S70, the display control unit 11G refers to the respective number concentrations of the material components calculated in step S60 and displays the number concentrations of the material components, that is, urine material component concentrations, at the display unit 16. More specifically, when urine qualitative inspection results of the specimen associated with the specimen ID acquired in step S10 have been memorized at the server 35, the display control unit 11G acquires the urine qualitative inspection results for the acquired specimen ID from the server 35. When the urine qualitative inspection results for the acquired specimen ID are acquired, the display control unit 11G displays the urine material component concentrations for the acquired specimen ID and the urine qualitative inspection results for the acquired specimen ID in, for example, an inspection results screen 64 as shown in Fig. 8. Alternatively, when urine qualitative inspection results for the acquired specimen ID have not been memorized at the server 35, the display control unit 11G displays only the urine material component concentrations for the acquired specimen ID in the inspection results screen 64.

Fig. 8 is a view showing an example of the inspection results screen 64 displaying urine material component concentrations and urine qualitative inspection results. In the inspection results screen 64 in Fig. 8, the table at the left side displays the urine qualitative inspection results and the table at the right side displays the urine material component concentrations. The inspection results screen 64 is displayed as a pop-up over a base screen (not shown in the drawings).

The inspection results screen 64 displays a selection button area 6 constituted with an approval button (Approve) 6A, a review button (Review) 6B, a display button (Detail) 6C, and an end button (Close) 6D.

The approval button 6A is a button for approving the urine material component concentrations displayed in the inspection results screen 64, that is, the measurement results of the urine material component concentrations for the acquired specimen ID. Approving the urine material component concentrations fixes the measurement results of the urine material component concentrations for the acquired specimen ID.

The review button 6B is a button for recalculating the urine material component concentrations for the acquired specimen ID. A user selects the review button when the urine material component concentrations displayed in the inspection results screen 64 differ from urine material component concentrations estimated from the urine qualitative inspection results or the like, or the user wishes to calculate the urine material component concentrations in more detail. When the review button 6B is selected, the user wishes to recalculate the urine material component concentrations in the acquired specimen. Hence, the control unit 10A sends the material component images 3 obtained from the acquired specimen together with the specimen ID to the data management device 20, which provides a service of classifying the material component images 3. In accordance with an instruction from the user, the control unit 10A also sends information other than the specimen ID and the material component images 3 to the data management device 20. For example, the control unit 10A sends to the data management device 20 the specimen ID and material component images 3 of the acquired specimen, the classification list that associates the respective material component images 3 of the acquired specimen with the types of material components (see Table 2), and the measurement results of the urine material component concentrations of the types of material components contained in the acquired specimen.

The display button 6C is a button for displaying the material component images 3 for the acquired specimen ID that are used in the calculation of a urine material component concentration. A user selects the display button 6C when the user wishes to check a material component included with the acquired specimen ID.

The end button 6D is a button for closing the inspection results screen 64 and displaying the base screen (not shown in the drawings).

In step S80 in Fig. 6, the reception unit 11E makes a determination as to whether a material component image display instruction has been received from the user. More specifically, the control unit 10A makes a determination as to whether, for example, the display button 6C of the inspection results screen 64 shown in Fig. 8 has been pressed. When the control unit 10A determines that the display button 6C has been pressed (the result of the determination is affirmative), the CPU 11 proceeds to step S90, and when the control unit 10A determines that the display button 6C has not been pressed (the result of the determination is negative), the CPU 11 proceeds to step S100.

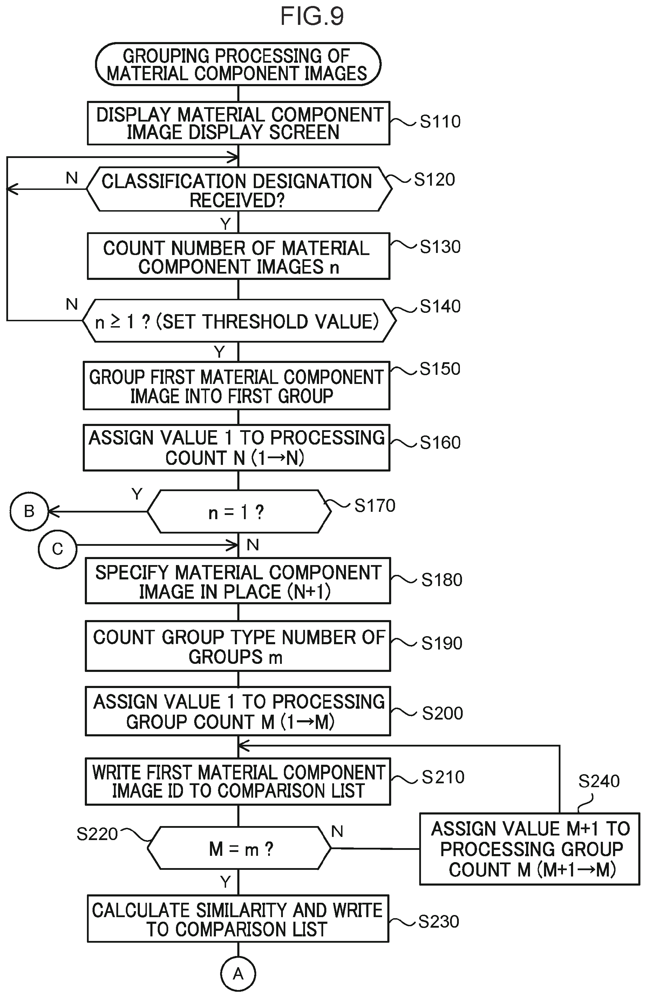

In step S90, the control unit 10A performs grouping processing of the material component images. Below, the grouping processing of material component images is more specifically described with reference to Fig. 9 to Fig. 14.

Fig. 9 is a flowchart showing an example of a flow of the grouping processing of material component images relating to step S90 in Fig. 6.

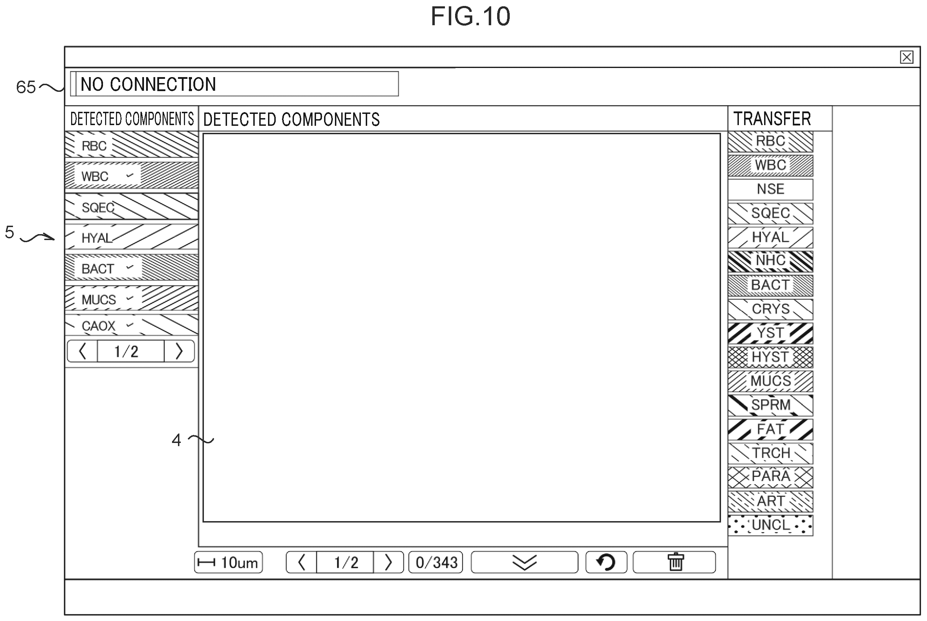

In step S110 of Fig. 9, the display control unit 11G displays, for example, a material component image display screen 65 as shown in Fig. 10 at the display unit 16.

Fig. 10 is a view showing an example of the material component image display screen 65 according to the present exemplary embodiment.

The material component image display screen 65 shown in Fig. 10 includes an image display region 4 and an item button group 5. The item button group 5 is buttons that are provided for each of the types of material component contained in the acquired specimen. In the image display region 4, the display control unit 11G displays the material component images 3 of a type of material component associated with a button that is designated by the user in the item button group 5. The image display region 4 displays the material component images 3 that are selected from groups as representative images.

In step S120 of Fig. 9, the reception unit 11E makes a determination as to whether a classification designation has been received from a user in the material component image display screen 65 illustrated in Fig. 10. When the reception unit 11E determines that a classification designation has been received (the result of the determination is affirmative), the CPU 11 proceeds to step S130, and when the reception unit 11E determines that no classification designation has been received (the result of the determination is negative), the CPU 11 proceeds to step S120. In the present example, a case is illustrated in which, as an example, the button "RBC" (red blood cells) in the item button group 5 is pressed by the user and "red blood cells" is designated.

In step S130, the grouping unit 11F counts a number n of the material component images associated with the classification designated in step S120. In the present example, the same as in the example in Fig. 5 described above, n is counted to be 4.

In step S140, the grouping unit 11F makes a determination as to whether n≥1. When the grouping unit 11F determines that n≥1 (the result of the determination is affirmative), the CPU 11 proceeds to step S150, and when the grouping unit 11F determines that n<1 (the result of the determination is negative), the CPU 11 returns to step S120 and repeats the processing. A threshold value is specified for judging similarities in this processing. The threshold value is set to a suitable value based on previous findings and experimental results. The threshold value may be a preset, and may be entered and changed as appropriate by users.

In step S150, the grouping unit 11F divides out the material component image associated with a material component image ID that is first, in a sequence of the material component image IDs associated with the classification designated in step S120 arranged by a predetermined condition (for example, numerical order), to a first group. More specifically, as illustrated in Table 4, a group field is added to the classification list in Table 2 described above, and a "1" indicating the first group is written in this group field. In the present example, of the material component image IDs #B00001, #B00003, #B00004 and #B00005 classified to the classification "red blood cells" designated by the user, the material component image corresponding to #B00001, in which the number contained in the material component image ID is smallest, is divided out to the first group.

**Table 4**

| Specimen ID | Material component image ID | Classification | Group |
|---|---|---|---|
| #A0002 | #B00001 | Red blood cells | 1 |
| #A0002 | #B00002 | White blood cells | |
| #A0002 | #B00003 | Red blood cells | |
| #A0002 | #B00004 | Red blood cells | |
| #A0002 | #B00005 | Red blood cells | |
| : | : | : | : |
| #A0002 | #B17904 | Squamous epithelial cells | |
| #A0002 | #B17905 | Bacteria | |

In step S160, the grouping unit 11F assigns the value 1 to the processing count N (1→N).

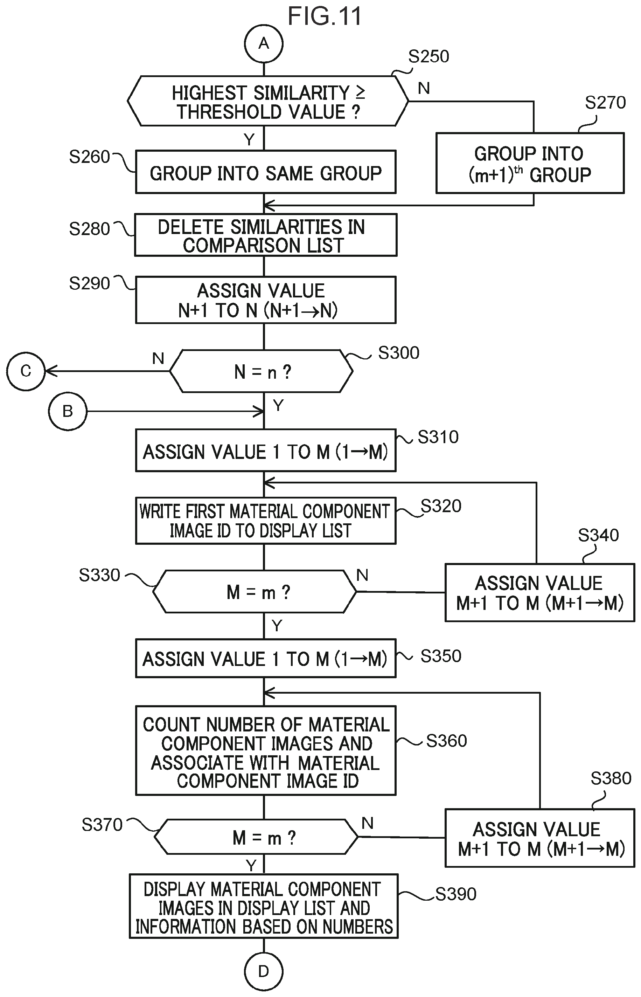

In step S170, the grouping unit 11F makes a determination as to whether n equals 1. When the grouping unit 11F determines that n equals 1 (the result of the determination is affirmative), the CPU 11 proceeds to step S310 in Fig. 11 and the grouping processing ends. When the grouping unit 11F determines that n does not equal 1 (the result of the determination is negative), the CPU 11 proceeds to step S180.

In step S180, the grouping unit 11F specifies the material component image associated with the material component image ID that is in place (N+1) from the start of the sequence arranged by the predetermined condition (in the example, numerical order) of the material component image IDs associated with the classification designated in step S120. In the present example, because N is 1, place (N+1) is second and #B00003, the numerical part of which is second smallest among the material component image IDs #B00001, #B00003, #B00004 and #B00005, is specified as this material component image ID.

In step S190, the grouping unit 11F counts the group type number m of groups associated with the material component image IDs associated with the classification designated in step S120. In the present example, because the first group exists in the classification list in Table 4, m equals 1.

In step S200, the grouping unit 11F assigns the value 1 to the processing group count M (1→M).

In step S210, the grouping unit 11F selects the material component image ID that is first in a sequence of the material component image IDs associated with the M^{th} group arranged by a predetermined condition (for example, numerical order), and writes this material component image ID to the comparison list. An example of the comparison list is illustrated in Table 5. In the present example, the grouping unit 11F selects #B00001 from the first group and writes #B00001 to the comparison list.

**Table 5**

| Material component image ID | Group |
|---|---|
| #B00001 | 1 |

In step S220, the grouping unit 11F makes a determination as to whether M equals m. When the grouping unit 11F determines that M equals m (the result of the determination is affirmative), the CPU 11 proceeds to step S230. When the grouping unit 11F determines that M does not equal m, that is, M is smaller than m (the result of the determination is negative), the CPU 11 proceeds to step S240.

In step S230, for each material component image corresponding with a material component image ID written in the comparison list of Table 5, the grouping unit 11F calculates a similarity between the respective material component image corresponding with the material component image ID written in the comparison list and the specified material component image corresponding with the material component image ID that is in place (N+1). The grouping unit 11F writes the calculated similarities to the comparison list. More specifically, as illustrated in Table 6, a similarity field is added to the comparison list in Table 5 described above, and the calculated similarities are written to the similarity field. The similarity in Table 6 is displayed as a percentage.

**Table 6**

| Material component image ID | Group | Similarity |
|---|---|---|
| #B00001 | 1 | 60 |

Alternatively, in step S240 the grouping unit 11F assigns the value M+1 to M (M+1→M), returns to step S210, selects the material component image ID that is first in a sequence of the material component image IDs associated with the M^{th} group arranged by the predetermined condition (in the example, numerical order), and writes this material component image ID to the comparison list.

Fig. 11 is a flowchart, continuing from Fig. 9, showing the example of the flow of grouping processing of material component images relating to step S90 in Fig. 6.

In step S250 of Fig. 11, when M is equal to m, the grouping unit 11F makes a determination as to whether the highest similarity among the similarities written to the comparison list is at least the threshold value. When the highest similarity is at least the threshold value (when the result of the determination is affirmative), the CPU 11 proceeds to step S260, and when the highest similarity is less than the threshold value (when the result of the determination is negative), the CPU 11 proceeds to step S270.

In step S260, the grouping unit 11F divides out the material component image corresponding with the specified material component image ID that is in place (N+1) to the same group as the group to which the material component image presenting the highest similarity has been divided out.

Alternatively, in step S270 the grouping unit 11F divides out the material component image corresponding with the specified material component image ID that is in place (N+1) to an (m+1)^{th} group. For example, when N equals 1 and m equals 1, as illustrated in the classification list in Table 7, the material component image corresponding with #B00003, which is second, is divided out to the second group.

**Table 7**

| Specimen ID | Material component image ID | Classification | Group |
|---|---|---|---|
| #A0002 | #B00001 | Red blood cells | 1 |
| #A0002 | #B00002 | White blood cells | |
| #A0002 | #B00003 | Red blood cells | 2 |
| #A0002 | #B00004 | Red blood cells | |
| #A0002 | #B00005 | Red blood cells | |
| : | : | : | : |
| #A0002 | #B17904 | Squamous epithelial cells | |
| #A0002 | #B17905 | Bacteria | |

In step S280, for grouping of the next material component image, the grouping unit 11F deletes the similarities in the comparison list.

In step S290, the grouping unit 11F assigns the value N+1 to N (N+1→N).

In step S300, the grouping unit 11F makes a determination as to whether N equals n. When the grouping unit 11F determines that N equals n (the result of the determination is affirmative), the CPU 11 proceeds to step S310. When the grouping unit 11F determines that N does not equal n, that is, N is smaller than n, (the result of the determination is negative), the CPU 11 returns to step S180 in Fig. 9 and repeats the processing to specify the material component image corresponding with the material component image ID that is in place (N+1) from the start of the sequence. When N is equal to n at step S300, the grouping of material component images ends. An example of a classification list when the grouping has ended is illustrated in Table 8.

**Table 8**

| Specimen ID | Material component image ID | Classification | Group |
|---|---|---|---|
| #A0002 | #B00001 | Red blood cells | 1 |
| #A0002 | #B00002 | White blood cells | |
| #A0002 | #B00003 | Red blood cells | 2 |
| #A0002 | #B00004 | Red blood cells | 3 |
| #A0002 | #B00005 | Red blood cells | 1 |
| : | : | : | : |
| #A0002 | #B17904 | Squamous epithelial cells | |
| #A0002 | #B17905 | Bacteria | |

In step S310 the display control unit 11G assigns the value 1 to M (1→M).

In step S320, the display control unit 11G selects the material component image ID that is first in the sequence of the material component image IDs associated with the M^{th} group arranged by the predetermined condition (in the example, numerical order), and writes this material component image ID to a display list.

In step S330, the display control unit 11G makes a determination as to whether M equals m. When the grouping unit 11F determines that M does not equal m, that is, M is smaller than m (the result of the determination is negative), the CPU 11 proceeds to step S340. When the display control unit 11G determines that M equals m (the result of the determination is affirmative), the CPU 11 proceeds to step S350.

In step S340, the display control unit 11G assigns the value M+1 to M (M+1→M), returns to step S320, selects the material component image ID that is first in the sequence arranged by the predetermined condition (in the example, numerical order) of the material component image IDs associated with the M^{th} group, and writes this material component image ID to the display list. The material component image ID that is written to the display list at this time is the material component image ID of the representative image of the M^{th} group. An example of a display list that is finally obtained is illustrated in Table 9.

**Table 9**

| Material component image ID | Group |
|---|---|
| #B00001 | 1 |
| #B00003 | 2 |
| #B00004 | 3 |

In step S350, when M equals m, the display control unit 11G assigns the value 1 to M (1→M).

In step S360, the display control unit 11G counts the number of material component images associated with the M^{th} group, and associates the counted number with the material component image ID that is associated with the M^{th} group in the display list.

In step S370, the display control unit 11G makes a determination as to whether M equals m. When the display control unit 11G determines that M does not equal m, that is, M is smaller than m (the result of the determination is negative), the CPU 11 proceeds to step S380. When the display control unit 11G determines that M equals m (the result of the determination is affirmative), the CPU 11 proceeds to step S390.

In step S380, the display control unit 11G assigns the value M+1 to M (M+1→M), returns to step S360, counts the number of material component images associated with the M^{th} group, and associates the counted number with the material component image ID that is associated with the M^{th} group in the display list. More specifically, as illustrated in Table 10, an image count field is added to the display list of Table 9 described above, and the counted numbers are written to the image count field.

**Table 10**

| Material component image ID | Group | Image count |
|---|---|---|
| #B00001 | 1 | 2 |
| #B00003 | 2 | 1 |
| #B00004 | 3 | 1 |

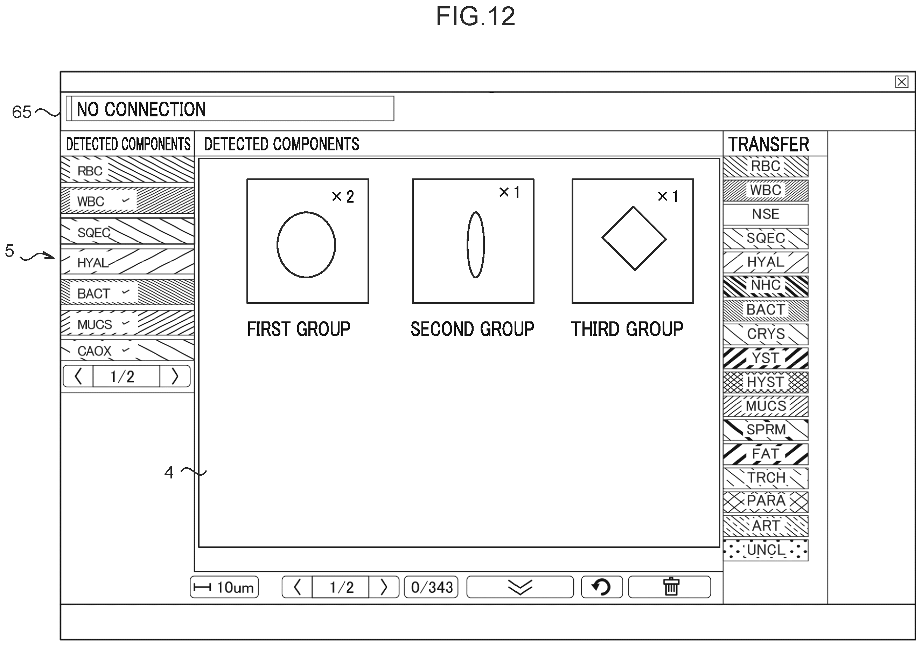

In step S390, when M is equal to m, the display control unit 11G displays at the display unit 16 the material component images associated with the material component image IDs in the display list and the image counts associated with those material component image IDs, that is, the representative images of the groups and information based on the image counts, for example, as illustrated in Fig. 12.

Fig. 12 is a view showing an example of the material component image display screen 65 according to the present exemplary embodiment in the state in which the representative images of the groups and the information based on the image counts of the groups are displayed.

Representative images representing the groups of material component images are displayed in the image display region 4 of the material component image display screen 65 illustrated in Fig. 12. R The representative images referred to here are, for example, the material component images corresponding with the material component image IDs that are arranged first in each group. In the example in Fig. 12, from the display list in Table 10, the material component image corresponding with #B00001 is displayed as the representative image of the first group, the material component image corresponding with #B00003 is displayed as the representative image of the second group, and the material component image corresponding with #B00004 is displayed as the representative image of the third group. In the respective upper rights of the representative images, "x2", "x1" and "x1" are displayed. These labels "x2", "x1" and "x1" represent the numbers of material component images belonging to the groups.

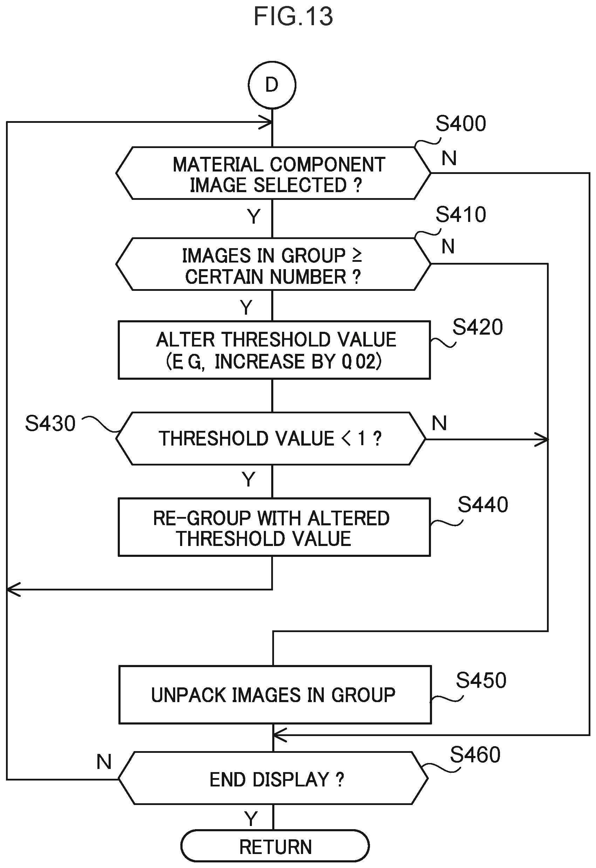

Fig. 13 is a flowchart, continuing from Fig. 11, showing the example of the flow of grouping processing of material component images relating to step S90 in Fig. 6.

In step S400 of Fig. 13, the display control unit 11G makes a determination as to whether, for example, a material component image that is a representative image of a group in the material component image display screen 65 of Fig. 12 has been selected by the user. When the display control unit 11G determines that a material component image has been selected by the user (when the result of the determination is affirmative), the CPU 11 proceeds to step S410, and when the display control unit 11G determines that no material component image has been selected by the user (when the result of the determination is negative), the CPU 11 proceeds to step S460.

In step S410, the display control unit 11G makes a determination as to whether the number of images in the group to which the material component image selected at step S400 belongs is at least a certain number. When the display control unit 11G determines that at least the certain number of images are in the group (when the result of the determination is affirmative), the CPU 11 proceeds to step S420, and when the display control unit 11G determines that less than the certain number of images are in the group (when the result of the determination is negative), the CPU 11 proceeds to step S450.

In step S420, the grouping unit 11F alters the threshold value. For example, the grouping unit 11F increases the current threshold value by 0.02. Thus, the threshold value may be specified in accordance with the number of material component images divided out to a group. When at least the certain number of images are in a group, which is to say, there are too many images in the group, the threshold value may have been specified to be lower than a suitable value, enabling the inclusion of images that are not particularly similar. Accordingly, increasing the threshold value is desirable for excluding images that are not similar.

In step S430, the grouping unit 11F makes a determination as to whether the altered threshold value is less than 1. When the grouping unit 11F determines that the altered threshold value is less than 1 (when the result of the determination is affirmative), the CPU 11 proceeds to step S440, and when the grouping unit 11F determines that the altered threshold value is 1 or greater (when the result of the determination is negative), the CPU 11 proceeds to step S450. In the present exemplary embodiment, the determination is whether the threshold value is less than 1, but the value that the threshold value is compared with may be suitably altered in accordance with a type of image classification that is being used for outputting compatibilities, or the like.

In step S440, The grouping unit 11F returns to step S140 in Fig. 9 and sets the altered threshold value. By processing the same as the processing from step S140 to step S370, the CPU 11 re-groups the images in the group to which the material component image selected at step S400 belongs and displays, at the display unit 16, representative images of groups determined by the re-grouping and numbers of images belonging to the groups determined by the re-grouping to which the representative images belong. Thereafter, the CPU 11 proceeds to step S460.

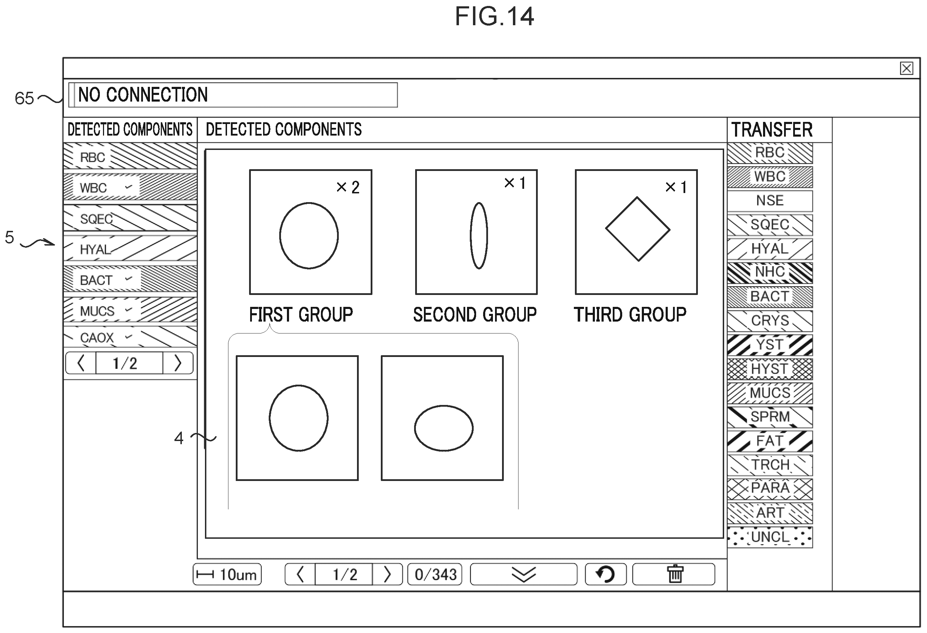

In step S450, the display control unit 11G unpacks the images in a group. That is, when an instruction selecting a representative image is received from the user, the display control unit 11G displays the material component images included in the group to which that representative image belongs at the display unit 16, for example, as shown in Fig. 14.

Fig. 14 is a view showing an example of the material component image display screen 65 according to the present exemplary embodiment in a state in which material component images in a group are displayed.

Representative images representing the groups of material component images, as described above, are displayed in the image display region 4 of the material component image display screen 65 illustrated in Fig. 14. When, for example, the certain number in step S410 is 10 and the user performs an operation to select the representative image of the first group, the number of images in the first group is 2. Because 2 is smaller than 10, the CPU 11 proceeds from step S410 to step S450 and displays all of the material component images included in the first group. If all of the material component images included in the first group cannot be displayed in the display space, some of the material component images included in the first group may be selectively displayed, or display of all of the material component images by scrolling may be enabled. When the user performs an operation to select the representative image again, the representative image alone is displayed.

In step S460 of Fig. 13, the display control unit 11G makes a determination as to whether the display of material component images is ending. When the display control unit 11G determines that the display is not ending (when the result of the determination is negative), the display control unit 11G returns to step S400 and repeats the processing, and when the display control unit 11G determines that the display is ending (when the result of the determination is affirmative), the display control unit 11G returns to step S70 in Fig. 6.

Returning now to Fig. 6, in step S100 the control unit 10A makes a determination as to whether an instruction to end measurement has been received. When the control unit 10A determines that an instruction to end measurement has not been received (when the result of the determination is negative), the control unit 10A returns to step S70 and repeats the processing, and when the control unit 10A determines that an instruction to end measurement has been received (when the result of the determination is affirmative), the present sequence of processing by the information processing program 15A ends.

In a urine sedimentation inspection, large numbers of material component images capturing similar shapes, principally of bacteria, crystals, white blood cells, red blood cells and fungi, are inspected, but a lot of time is required to examine material component images one by one. In contrast, according to the present exemplary embodiment, the material component images are grouped and only the representative images are displayed. Therefore, examination of the images may be conducted efficiently.

Automatic classification results may include misclassifications. Many systems are equipped with processes for manual correction. However, if all similar images are displayed, a lot of time is required to find misclassified images in a large number of images. In contrast, according to the present exemplary embodiment, because similar material component images are grouped and representative images of groups are presented to the user, misclassified images may be found efficiently.

If large numbers of similar images are successively displayed, there is a high likelihood of images that capture peculiar shapes being overlooked. However, peculiar shapes that differ from typical shapes, for example, mulberry bodies, heteromorphic cells, glomerular red blood cells and the like, may have clinical significance. According to the present exemplary embodiment, because similar material component images are grouped, images in which peculiar shapes are captured are less likely to be overlooked.

In the present exemplary embodiment, only the representative images of the groups into which the material component images are grouped are displayed. When a representative image is selected, the material component images classified into the group to which the selected representative image belongs are unpacked and displayed. As an alternative exemplary embodiment, a number of types of group in the classification list in Table 8 that are created by the processing of step S130 to step S300 is counted, a number of display fields the same as the number of types of group are displayed at the display unit 16, and the material component images of the respective groups may be displayed side by side in the respective display fields. For example, when the material component images classified as "red blood cells" are divided into groups of three types as in Table 8, three fields are provided in an upper area, a middle area and a lower area of the image display region 4 of the display unit 16. The material component image of #B00001 and the material component image of #B00005 that are divided out to the first group are displayed side by side in the field in the upper area, the material component image of #B00003 that is divided out to the second group is displayed in the field in the middle area, and the material component image of #B00004 that is divided out to the third group is displayed in the field in the lower area.

### = Second Exemplary Embodiment =

In the first exemplary embodiment described above, a mode is described in which the information processing device constitutes part of the urine material component analysis device. In the second exemplary embodiment, a mode is described that is structured as a system including a portable terminal and the information processing device.

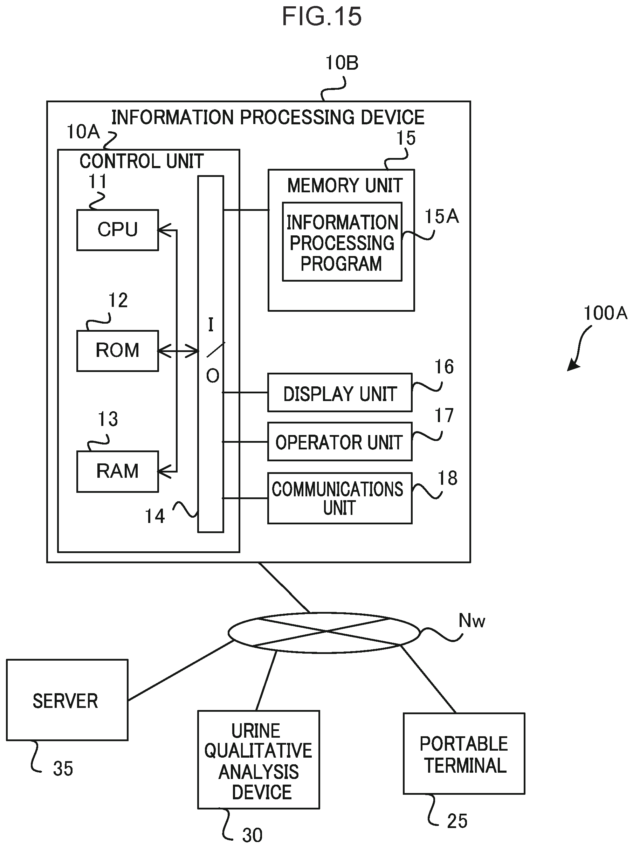

Fig. 15 is a block diagram showing an example of electronic structures of an information processing system 100A according to the second exemplary embodiment.

As shown in Fig. 15, the information processing system 100A according to the present exemplary embodiment includes an information processing device 10B, a portable terminal 25, the urine qualitative analysis device 30 and the server 35. The information processing device 10B is connected with each of the portable terminal 25 and the urine qualitative analysis device 30 via the network circuit Nw. The urine qualitative analysis device 30 is connected to the server 35 via the network circuit Nw. The urine qualitative analysis device 30 is linked with, for example, the urine material component analysis device 70.

A general-purpose computer apparatus such as a server computer or the like is employed for the information processing device 10B according to the present exemplary embodiment. The information processing device 10B is structured separately from the urine material component analysis device 70. Structures of the information processing device 10B are similar to the information processing device 10 according to the first exemplary embodiment.

A portable computer apparatus such as, for example, a smartphone, a tablet terminal or the like is employed for the portable terminal 25. The portable terminal 25 is equipped with a camera capable of imaging a specimen. The portable terminal 25 sends specimen images capturing a specimen or material component images extracted from specimen images to the information processing device 10B.

The information processing device 10B acquires material component images from the portable terminal 25. When the information processing device 10B receives a specimen image from the portable terminal 25, the information processing device 10B acquires material component images by extraction from the specimen image. The information processing device 10B performs classification processing and grouping processing on the acquired material component images, and sends the processing results to the portable terminal 25.

When the information processing device 10B receives, for example, a specimen image or material component images from the portable terminal 25, the information processing device 10B sends the material component image IDs, classifications, and representative images associated with groups to the portable terminal 25. In this case, the portable terminal 25 only carries out processing to display the representative images. When the information processing device 10B receives material component images from the portable terminal 25, the information processing device 10B may send only material component image IDs, classifications and grouping information relating to groups to the portable terminal 25. In this case, the portable terminal 25 carries out processing to specify and display representative images on the basis of the grouping information. The portable terminal 25 may have functions for associating material component image IDs with material component images.

According to the present exemplary embodiment as described above, the information processing device may acquire a specimen image or material component images from a portable terminal, the material component images may be grouped at the information processing device, and the representative images of the groups may be displayed at the portable terminal.

The term "processor" as used in the exemplary embodiments described above refers to a processor in a broad sense, encompassing general purpose processors (for example, a central processing unit (CPU) or the like), dedicated processors (for example, a graphics processing unit (GPU), application-specific integrated circuit (ASIC), field programmable gate array (FPGA), programmable logic device or the like) and so forth.

Operations of the processor in the exemplary embodiments described above need not be conducted by a single processor but may be conducted by plural processors at physically distant locations in co-ordination. Furthermore, an order of operations of the processor is not limited to the order recited in the above exemplary embodiments and may be modified as appropriate.

Above, the information processing device 10 according to an exemplary embodiment is described as an example. The exemplary embodiment may be embodied as a program for causing a computer to realize the functions of the units provided at the information processing device 10. The exemplary embodiment may also be embodied as a computer-readable non-transitory recording medium memorizing the program.

In addition, structures of the information processing device 10 in the exemplary embodiments described above are examples, and may be modified in accordance with circumstances within a scope not departing from the gist of the disclosure. Displays of the material component images 3 are not limited by the exemplary embodiments described above but may be displays, for example, in left-to-right rows. Display positions of the buttons may also be modified as appropriate.

The flow of the processing of the program described in the above exemplary embodiments is an example. Unnecessary steps may be removed, new steps may be added, and the processing sequence may be rearranged within a scope not departing from the gist of the disclosure.

In the exemplary embodiments described above, configurations are described in which processing relating to the exemplary embodiments is implemented by a software configuration using a computer, by executing a program, but this is not limiting. The exemplary embodiments may implement the processing by, for example, a hardware configuration or a combination of a hardware configuration and a software configuration.

The following supplementary notes are disclosed in relation to the exemplary embodiments described above.

### - Supplementary note 1 -

An information processing device includes:
an acquisition unit that acquires material component images in which material components contained in a specimen are captured;
a classification unit that classifies the material component images based on types of the material components captured in the material component images;
a reception unit that receives a designation of a classification from a user;
a grouping unit that divides out the material component images that are classified with the classification designated at the reception unit into groups one by one in order, the grouping unit:
   sequentially reading the material component images classified with the designated classification,
   memorizing a material component image that is read first as a representative image of a first group of the classification,
   comparing a material component image that is read next with the representative image of the first group,
   in a case in which this material component image and the representative image attain a predetermined similarity, memorizing this material component image in the first group,
   in a case in which this material component image and the representative image are not similar, memorizing this material component image as a representative image of a new group that is separate from the first group,
   comparing a material component image that is sequentially read third or subsequently with the memorized representative image of each group,
   in a case in which this material component image is similar to any of the representative images, grouping this material component image with a group whose representative image is similar, and
   in a case in which this material component image is not similar to any of the representative images, memorizing this material component image as a representative image of a new group; and
   a display control unit that performs control to display, at a display unit, the representative image of the material component images in each group divided by the grouping unit.

### - Supplementary note 2 -

The information processing device according to supplementary note 1 further includes:
an imparting unit that imparts identification information for identifying the material component images, to the material component images acquired by the acquisition unit; and
an association unit that associates the classifications of material components with the material component images to which the identification information is imparted,
wherein
the reception unit receives from the user, the designation of a classification among the classifications associated with the material component images,
if a number of material component images associated with the designated classification is represented by n, a processing count is represented by N, a number of types of groups associated with the identification information associated with the designated classification is m, and a processing group count is represented by M, the grouping unit:
   arranges the identification information associated with the designated classification in sequence by a predetermined condition and, while incrementing N from 1 to (n-1) in increments of 1, specifies the material component image corresponding with the identification information that is in each place (N+1) from a start of the sequence,
   while incrementing M from 1 to m in increments of 1, arranges the identification information associated with each M^{th} group in sequence by a predetermined condition, and selects the identification information that is in first place in this sequence,
   in a case in which M is equal to m, for each material component image corresponding with the selected identification information, calculates a similarity between the each material component image corresponding with the selected identification information and the specified material component image corresponding with the identification information in place (N+1),
   in a case in which a highest similarity among the calculated similarities is at least a threshold value, divides out the specified material component image corresponding with the identification information in place (N+1) to the same group as a group to which the material component image presenting the highest similarity has been divided out, and
   in a case in which the highest similarity among the calculated similarities is less than the threshold value, divides out the specified material component image corresponding with the identification information in place (N+1) to an (m+1)^{th} group; and
   the display control unit performs control to display at the display unit, as the representative image of the material component images in each group divided by the grouping unit, the material component image corresponding with the identification information that is first in the group.

### - Supplementary note 3 -

In the information processing device according to supplementary note 2, when an operation on one of the representative images is received, the display control unit performs control to display, at the display unit, the material component images included in the group to which the representative image belongs.

### - Supplementary note 4 -

In the information processing device according to supplementary note 2 or supplementary note 3, the grouping unit is capable of specifying the threshold value in accordance with a number of the material component images divided out to the groups.

### - Supplementary note 5 -

In the information processing device according to any one of supplementary notes 2 to 4, the identification information is material component image IDs that are determined based on an order in which the material component images are extracted from a captured image.

### - Supplementary note 6 -

In the information processing device according to supplementary note 5, the grouping unit:
counts the number n of the material component images associated with the designated classification;
arranges the material component image IDs associated with the designated classification in sequence by the predetermined condition, and divides out the material component image corresponding with the material component image ID that is first in the sequence to a first group;
assigns a value 1 to the processing count N;
specifies the material component image corresponding with the material component image ID that is in place (N+1) from a start of the sequence of the material component image IDs associated with the designated classification arranged by the predetermined condition;
counts the group type number m of the groups associated with the material component image IDs associated with the designated classification;
assigns the value 1 to the processing group count M;
arranges the material component image IDs associated with the M^{th} group in sequence by the predetermined condition, selects the material component image ID that is first in this sequence, and writes this material component image ID to a comparison list;
in a case in which M is less than m, assigns a value M+1 to M, and returns to the processing that arranges the material component image IDs associated with the M^{th} group in sequence by the predetermined condition, selects the material component image ID that is first in this sequence, and writes this material component image ID to the comparison list;
in a case in which M is equal to m, for each material component image corresponding with the material component image IDs written to the comparison list, calculates a similarity between the each material component image corresponding with the material component image ID written to the comparison list and the specified material component image corresponding with the material component image ID that is in place (N+1), and writes the calculated similarity to the comparison list;
in a case in which a highest similarity among the similarities written to the comparison list is at least the threshold value, divides out the specified material component image corresponding with the material component image ID that is in place (N+1) to the same group as a group to which the material component image presenting the highest similarity has been divided out;
in a case in which the highest similarity among the similarities written to the comparison list is less than the threshold value, divides out the specified material component image corresponding with the component image ID that is in place (N+1) to an (m+1)^{th} group;
assigns a value N+1 to the processing count N;
in a case in which N is less than n, returns to the processing that specifies the material component image corresponding with the material component image ID that is in place (N+1) from the start of the sequence of the material component image IDs associated with the designated classification arranged by the predetermined condition; and
in a case in which N is equal to n, ends the grouping of the material component images associated with the designated classification.

### - Supplementary note 7 -

In the information processing device according to supplementary note 6, the display control unit:
assigns the value 1 to M, arranges the material component image IDs associated with the M^{th} group in sequence by the predetermined condition, selects the material component image ID that is first in the sequence, and writes this material component image ID to a display list;
in a case in which M is smaller than m, assigns the value M+1 to M, and returns to the processing that arranges the material component image IDs associated with the M^{th} group in sequence by the predetermined condition, selects the material component image ID that is first in this sequence, and writes this material component image ID to the display list;
in a case in which M is equal to m, assigns the value 1 to M, counts a number of material component images associated with the M^{th} group, and associates the counted number with the material component image ID that is associated with the M^{th} group in the display list;
in a case in which M is smaller than m, assigns the value M+1 to M, and returns to the processing that counts the number of material component images associated with the M^{th} group and associates the counted number with the material component image ID that is associated with the M^{th} group in the display list; and
in a case in which M is equal to m, performs control to display at the display unit the material component images corresponding with the material component image IDs in the display list and information based on the numbers associated with these material component image IDs.

### - Supplementary note 8 -

An analysis device includes:
a flow cell through which a specimen containing material components flows;
an imaging unit that images the specimen flowing through the flow cell;
the information processing device according to any one of supplementary notes 1 to 7, the information processing device being connected to the imaging unit; and
a display unit connected to the information processing device.

### - Supplementary note 9 -

An information processing method includes a computer executing processing including:
acquiring material component images in which material components contained in a specimen are captured;
classifying the material component images based on types of the material components captured in the material component images;
receiving a designation of a classification from a user;
dividing out the material component images that are classified with the designated classification, into groups one by one, in order, the dividing out including:
   sequentially reading the material component images classified with the designated classification,
   memorizing a material component image that is read first as a representative image of a first group of the classification,
   comparing a material component image that is read next with the representative image of the first group,
   in a case in which this material component image and the representative image attain a predetermined similarity, memorizing this material component image in the first group,
   in a case in which this material component image and the representative image are not similar, memorizing this material component image as a representative image of a new group that is separate from the first group,
   comparing a material component image that is sequentially read third or subsequently with the memorized representative image of each group,
   in a case in which this material component image is similar to any of the representative images, grouping this material component image with a group whose representative image is similar, and
   in a case in which this material component image is not similar to any of the representative images, memorizing this material component image as a representative image of a new group; and
   performing control to display at a display unit, the representative image of the material component images in each divided group.

### - Supplementary note 10 -

An information processing program causes a computer to execute processing including:
acquiring material component images in which material components contained in a specimen are captured;
classifying the material component images based on types of the material components captured in the material component images;
receiving a designation of a classification from a user;
dividing out the material component images that are classified with the designated classification into groups one by one, in order, the dividing out including:
   sequentially reading the material component images classified with the designated classification,
   memorizing a material component image that is read first as a representative image of a first group of the classification,
   comparing a material component image that is read next with the representative image of the first group,
   in a case in which this material component image and the representative image attain a predetermined similarity, memorizing this material component image in the first group,
   in a case in which this material component image and the representative image are not similar, memorizing this material component image as a representative image of a new group that is separate from the first group,
   comparing a material component image that is sequentially read third or subsequently with the memorized representative image of each group,
   in a case in which this material component image is similar to any of the representative images, grouping this material component image with a group whose representative image is similar, and
   in a case in which this material component image is not similar to any of the representative images, memorizing this material component image as a representative image of a new group; and
   performing control to display at a display unit, the representative image of the material component images in each divided group.

The disclosures of Japanese Patent Application No. 2023-177846 filed October 13, 2023 are incorporated into the present specification by reference in their entirety. All references, patent applications and technical specifications cited in the present specification are incorporated by reference into the present specification to the same extent as if the individual references, patent applications and technical specifications were specifically and individually recited as being incorporated by reference.

## Claims

1. An information processing device, comprising:
an acquisition unit that acquires material component images in which material components contained in a specimen are captured;
a classification unit that classifies the material component images based on types of the material components captured in the material component images;
a reception unit that receives a designation of a classification from a user;
a grouping unit that divides out the material component images that are classified with the classification designated at the reception unit, into groups one by one, in order, the grouping unit:
sequentially reading the material component images classified with the designated classification,
memorizing a material component image that is read first as a representative image of a first group of the classification,
comparing a material component image that is read next with the representative image of the first group,
in a case in which this material component image and the representative image attain a predetermined similarity, memorizing this material component image in the first group,
in a case in which this material component image and the representative image are not similar, memorizing this material component image as a representative image of a new group that is separate from the first group,
comparing a material component image that is sequentially read third or subsequently with the memorized representative image of each group,
in a case in which this material component image is similar to any of the representative images, grouping this material component image with a group whose representative image is similar, and
in a case in which this material component image is not similar to any of the representative images, memorizing this material component image as a representative image of a new group; and
a display control unit that performs control to display, at a display unit, the representative image of the material component images in each group divided by the grouping unit.

2. The information processing device according to claim 1, further comprising:
an imparting unit that imparts identification information, for identifying the material component images, to the material component images acquired by the acquisition unit; and
an association unit that associates the classifications of material components with the material component images to which the identification information is imparted,
wherein:
the reception unit receives, from the user, the designation of a classification among the classifications associated with the material component images;
if a number of the material component images associated with the designated classification is represented by n, a processing count is represented by N, a group type number of groups associated with the identification information associated with the designated classification is represented by m, and a processing group count is represented by M, the grouping unit:
arranges the identification information associated with the designated classification in sequence by a predetermined condition and, while incrementing N from 1 to (n-1) in increments of 1, specifies the material component image corresponding with the identification information that is in each place (N+1) from a start of the sequence,
while incrementing M from 1 to m in increments of 1, arranges the identification information associated with each M^{th} group in sequence by a predetermined condition, and selects the identification information that is in first place in this sequence,
in a case in which M is equal to m, for each material component image corresponding with the selected identification information, calculates a similarity between the each material component image corresponding with the selected identification information and the specified material component image corresponding with the identification information in place (N+1),
in a case in which a highest similarity among the calculated similarities is at least a threshold value, divides out the specified material component image corresponding with the identification information in place (N+1) to the same group as a group to which the material component image presenting the highest similarity has been divided out, and
in a case in which the highest similarity among the calculated similarities is less than the threshold value, divides out the specified material component image corresponding with the identification information in place (N+1) to an (m+1)^{th} group; and
the display control unit performs control to display, at the display unit, as the representative image of the material component images in each group divided by the grouping unit, the material component image corresponding with the identification information that is first in the group.

3. The information processing device according to claim 2, wherein, when an operation on one of the representative images is received, the display control unit performs control to display, at the display unit, the material component images included in the group to which the representative image belongs.

4. The information processing device according to claim 2, wherein the grouping unit is capable of specifying the threshold value in accordance with a number of the material component images divided out to the groups.

5. The information processing device according to claim 2, wherein the identification information is material component image IDs that are determined based on an order in which the material component images are extracted from a captured image.

6. The information processing device according to claim 5, wherein the grouping unit:
counts the number n of the material component images associated with the designated classification;
arranges the material component image IDs associated with the designated classification in sequence by the predetermined condition, and divides out the material component image corresponding with the material component image ID that is first in the sequence to a first group;
assigns a value 1 to the processing count N;
specifies the material component image corresponding with the material component image ID that is in place (N+1) from a start of the sequence of the material component image IDs associated with the designated classification arranged by the predetermined condition;
counts the group type number m of the groups associated with the material component image IDs associated with the designated classification;
assigns the value 1 to the processing group count M;
arranges the material component image IDs associated with the M^{th} group in sequence by the predetermined condition, selects the material component image ID that is first in this sequence, and writes this material component image ID to a comparison list;
in a case in which M is less than m, assigns a value M+1 to M, and returns to the processing that arranges the material component image IDs associated with the M^{th} group in sequence by the predetermined condition, selects the material component image ID that is first in this sequence, and writes this material component image ID to the comparison list;
in a case in which M is equal to m, for each material component image corresponding with the material component image IDs written to the comparison list, calculates a similarity between the each material component image corresponding with the material component image ID written to the comparison list and the specified material component image corresponding with the material component image ID that is in place (N+1), and writes the calculated similarity to the comparison list;
in a case in which a highest similarity among the similarities written to the comparison list is at least the threshold value, divides out the specified material component image corresponding with the material component image ID that is in place (N+1) to the same group as a group to which the material component image presenting the highest similarity has been divided out;
in a case in which the highest similarity among the similarities written to the comparison list is less than the threshold value, divides out the specified material component image corresponding with the component image ID that is in place (N+1) to an (m+1)^{th} group;
assigns a value N+1 to the processing count N;
in a case in which N is less than n, returns to the processing that specifies the material component image corresponding with the material component image ID that is in place (N+1) from the start of the sequence of the material component image IDs associated with the designated classification arranged by the predetermined condition; and
in a case in which N is equal to n, ends the grouping of the material component images associated with the designated classification.

7. The information processing device according to claim 6, wherein the display control unit:
assigns the value 1 to M, arranges the material component image IDs associated with the M^{th} group in sequence by the predetermined condition, selects the material component image ID that is first in the sequence, and writes this material component image ID to a display list;
in a case in which M is smaller than m, assigns the value M+1 to M, and returns to the processing that arranges the material component image IDs associated with the M^{th} group in sequence by the predetermined condition, selects the material component image ID that is first in this sequence, and writes this material component image ID to the display list;
in a case in which M is equal to m, assigns the value 1 to M, counts a number of material component images associated with the M^{th} group, and associates the counted number with the material component image ID that is associated with the M^{th} group in the display list;
in a case in which M is smaller than m, assigns the value M+1 to M, and returns to the processing that counts the number of material component images associated with the M^{th} group and associates the counted number with the material component image ID that is associated with the M^{th} group in the display list; and
in a case in which M is equal to m, performs control to display, at the display unit, the material component images corresponding with the material component image IDs in the display list and information based on the numbers associated with these material component image IDs.

8. An analysis device, comprising:
a flow cell through which a specimen containing material components flows;
an imaging unit that images the specimen flowing through the flow cell;
the information processing device according to any one of claims 1 to 7, the information processing device being connected to the imaging unit; and
a display unit connected to the information processing device.

9. An information processing method comprising a computer executing processing including:
acquiring material component images in which material components contained in a specimen are captured;
classifying the material component images based on types of the material components captured in the material component images;
receiving a designation of a classification from a user;
dividing out the material component images that are classified with the designated classification, into groups one by one, in order, the dividing out including:
sequentially reading the material component images classified with the designated classification,
memorizing a material component image that is read first as a representative image of a first group of the classification,
comparing a material component image that is read next with the representative image of the first group,
in a case in which this material component image and the representative image attain a predetermined similarity, memorizing this material component image in the first group,
in a case in which this material component image and the representative image are not similar, memorizing this material component image as a representative image of a new group that is separate from the first group,
comparing a material component image that is sequentially read third or subsequently with the memorized representative image of each group,
in a case in which this material component image is similar to any of the representative images, grouping this material component image with a group whose representative image is similar, and
in a case in which this material component image is not similar to any of the representative images, memorizing this material component image as a representative image of a new group; and
performing control to display, at a display unit, the representative image of the material component images in each divided group.

10. An information processing program for causing a computer to execute processing comprising:
acquiring material component images in which material components contained in a specimen are captured;
classifying the material component images based on types of the material components captured in the material component images;
receiving a designation of a classification from a user;
dividing out the material component images that are classified with the designated classification, into groups one by one, in order, the dividing out including:
sequentially reading the material component images classified with the designated classification,
memorizing a material component image that is read first as a representative image of a first group of the classification,
comparing a material component image that is read next with the representative image of the first group,
in a case in which this material component image and the representative image attain a predetermined similarity, memorizing this material component image in the first group,
in a case in which this material component image and the representative image are not similar, memorizing this material component image as a representative image of a new group that is separate from the first group,
comparing a material component image that is sequentially read third or subsequently with the memorized representative image of each group,
in a case in which this material component image is similar to any of the representative images, grouping this material component image with a group whose representative image is similar, and
in a case in which this material component image is not similar to any of the representative images, memorizing this material component image as a representative image of a new group; and
performing control to display, at a display unit, the representative image of the material component images in each divided group.
